# EUROPEAN PATENT SPECIFICATION

(11) **EP 4 028 550 B1**
(45) Date of publication and mention of the grant of the patent: **06.11.2024**
(21) Application number: 20862385.0
(22) Date of filing: 04.09.2020
(51) Int. Cl.: C12Q 1/6855, C12Q 1/6853, C12Q 1/686, C12Q 1/6869, C12N 9/00

(54) **TARGETED SEQUENCING TO DETECT AND QUANTIFY LOW LEVELS OF METHYLATED DNA**
GEZIELTE SEQUENZIERUNG ZUM DETEKTIEREN UND QUANTIFIZIEREN VON NIEDRIGEN NIVEAUS METHYLIERTER DNA
SÉQUENÇAGE CIBLÉ POUR DÉTECTER ET QUANTIFIER DE FAIBLES TAUX D'ADN MÉTHYLÉ

(30) Priority: 09.09.2019 US 201962897814 P
(43) Date of publication of application: 20.07.2022
(73) Proprietor: University of Utah Research Foundation, Salt Lake City, UT 84108 (US)
(72) Inventor: VARLEY, Katherine E., Salt Lake City, Utah 84108 (US); MILLER, Ryan, Salt Lake City, Utah 84108 (US); GREENLAND, Jeffery A., Salt Lake City, Utah 84108 (US)
(74) Representative: Marks & Clerk LLP
(86) International application number: PCT/US2020/049528
(87) International publication number: WO 2021/050393

(56) References cited:
- WO-A1-2015/104302
- WO-A1-2018/031760
- WO-A1-2018/093780
- WO-A1-2019/126313
- US-A1- 2014 329 697
- VARLEY K. E., MITRA R. D.: "Bisulfite Patch PCR enables multiplexed sequencing of promoter methylation across cancer samples", GENOME RES., vol. 20, no. 9, September 2010 (2010-09-01), pages 1279 - 1287, XP055815221

## Description

### CROSS-REFERENCE TO RELATED APPLICATIONS

This application claims priority to U.S. Provisional Patent Application No. 62/897,814, filed September 9.

### STATEMENT REGARDING FEDERALLY SPONSORED RESEARCH

This invention was made with government support under grant CA204253 awarded by the National Institutes of Health. The government has certain rights in the invention.

### FIELD

This disclosure relates to targeted sequencing to detect and quantify low levels of methylated DNA in biological samples.

### INTRODUCTION

The detection and quantification of low levels of methylated DNA in biological samples has been difficult for several reasons. Many approaches treat DNA samples with chemicals or enzymes that convert the structure of the cytosine DNA base so that cytosine can be distinguished from 5-methyl-cytosine through DNA sequencing, however, the treatment of DNA samples to covert cytosine is damaging to the DNA, which limits the sensitivity of the method and results in false negatives when the methylated DNA is present at low levels (<1%). Furthermore, these treatments are not 100% efficient and specific, which results in false positives or false negatives that obscures detection of 5-methyl-cytosine present at low levels (<1%). Other conventional approaches use antibodies or proteins to physically enrich for methylated DNA, but the affinity of this approach is not specific enough to accurately quantify methylation present at low levels (<1 %). If the affinity purification assay is optimized for retention of all molecules containing 5-methyl-cytosine it results in false positives, and if the assay is optimized for exclusion of molecules that do not contain 5-methyl-cytosine, it results in false negatives. There is a need for accurate methods of detecting and quantifying low levels of methylated DNA.

### SUMMARY

In an aspect, the disclosure relates to a method of detecting methylation of cytosine residues in a target polynucleotide in a sample comprising DNA. The method may include digesting the DNA in the sample with a methyl-insensitive restriction enzyme to create a target polynucleotide comprising a plurality of cytosine residues, wherein one or more of the cytosine residues are methylated; ligating one end of the target polynucleotide to a Unique Molecular Identifier (UMI) polynucleotide and the other end of the target polynucleotide to a protective polynucleotide to form a ligated polynucleotide, wherein a portion of the UMI polynucleotide comprises a first randomly generated UMI polynucleotide sequence, wherein all of the cytosine residues of the UMI polynucleotide are methylated, and wherein the protective polynucleotide includes an exonuclease resistant moiety; contacting the sample with one or more exonucleases adapted to digest any polynucleotides in the sample that do not include the exonuclease resistant moiety; forming a converted polynucleotide by chemically and/or enzymatically converting each unmethylated cytosine in the ligated polynucleotide to uracil; amplifying the converted polynucleotide to generate a plurality of amplicon polynucleotides; sequencing the plurality of amplicon polynucleotides to generate a plurality of amplicon sequence reads, wherein each of the amplicon sequence reads: corresponds to the polynucleotide sequence of one of the plurality of amplicon polynucleotides; includes the randomly generated UMI polynucleotide sequence; and includes a thymine at each nucleotide position corresponding to the nucleotide position of an unmethylated cytosine in the target polynucleotide and a cytosine at each nucleotide position corresponding to a methylated cytosine in the target polynucleotide unless a conversion error during the conversion step, an amplification error during the amplification step, or a sequencing error during the sequencing step causes the amplicon sequence read to include a nucleotide other than thymine at a nucleotide position corresponding to the nucleotide position of an unmethylated cytosine in the target polynucleotide or to include a nucleotide other than cytosine at a nucleotide position corresponding to the nucleotide position of a methylated cytosine in the target polynucleotide; and aligning the plurality of amplicon sequencing reads with a target polynucleotide reference sequence; if the sequencing step generated at least five amplicon sequence reads, then generating a consensus polynucleotide sequence corresponding to the polynucleotide sequence of the target polynucleotide, wherein generating the consensus polynucleotide sequence comprises: identifying each 5'-C-G-3' nucleotide pair in the target polynucleotide reference sequence; comparing each amplicon sequencing read to the target polynucleotide reference sequence to determine the identity of each nucleotide in each amplicon sequencing read aligned with each cytosine in each 5'-C-G-3' nucleotide pair in the target polynucleotide reference sequence; if 50% or more of the amplicon sequencing reads include a cytosine at a position aligned with a cytosine in a 5'-C-G-3' nucleotide pair in the target polynucleotide reference sequence, then assigning a methylated cytosine to the corresponding position in the consensus polynucleotide sequence; and if less than 50% of the amplicon sequencing reads include a cytosine at a position aligned with a cytosine in a 5'-C-G-3' nucleotide pair in the target polynucleotide reference sequence, then assigning an unmethylated cytosine to the corresponding position in the consensus polynucleotide sequence; and wherein if at least 50% of the 5'-C-G-3' nucleotide pairs in the consensus polynucleotide sequence have been assigned a methylated cytosine, then designating the target polynucleotide as methylated, and wherein if less than 50% of the 5'-C-G-3' nucleotide pairs in the consensus polynucleotide sequence have been assigned a methylated cytosine, then designating the target polynucleotide as unmethylated.

In some embodiments, the method further includes calculating the fraction of methylated target polynucleotides in the sample by dividing the number of methylated target polynucleotides by the total number of target polynucleotides detected in the sample. In some embodiments, the exonuclease resistant modification comprises a phosphorothioate bond and/or a 3-carbon spacer. In some embodiments, ligating the target polynucleotide to the UMI polynucleotide comprises annealing a first patch polynucleotide to both the target polynucleotide and the UMI polynucleotide, and wherein ligating the target polynucleotide to the protective polynucleotide comprises annealing a second patch polynucleotide to both the target polynucleotide and the protective polynucleotide. In some embodiments, TET2 and APOBEC are used to enzymatically convert the ligated polynucleotide to the converted polynucleotide. In some embodiments, the converted polynucleotide is amplified using the polymerase chain reaction (PCR). In some embodiments, the target polynucleotide is from a region of a genome known to be methylated in a specific cell type. In some embodiments, the specific cell type is a specific type of cancer cell. In some embodiments, the specific type of cancer cell is a cancer cell selected from the group consisting of breast cancer, ovarian cancer, lung cancer, pancreatic cancer, colorectal cancer, prostate cancer, uterine cancer, bladder cancer, and liver cancer. In some embodiments, generating the consensus polynucleotide sequence comprises assigning a methylated cytosine to the corresponding position in the consensus polynucleotide sequence if 90% or more of the amplicon sequencing reads include a cytosine at a position aligned with a cytosine in a 5'-C-G-3' nucleotide pair in the target polynucleotide reference sequence, and assigning an unmethylated cytosine to the corresponding position in the consensus polynucleotide sequence if less than 90% of the amplicon sequencing reads include a cytosine at a position aligned with a cytosine in a 5'-C-G-3' nucleotide pair in the target polynucleotide reference sequence. In some embodiments, the target polynucleotide is designated as methylated if at least 75% of the 5'-C-G-3' nucleotide pairs in the consensus polynucleotide sequence have been assigned a methylated cytosine, and the target polynucleotide is designated as unmethylated if less than 75% of the 5'-C-G-3' nucleotide pairs in the consensus polynucleotide sequence have been assigned a methylated cytosine. In some embodiments, the consensus polynucleotide comprises a plurality of 5'-C-G-3' nucleotide pairs. In some embodiments, methylation of cytosine residues in a plurality of target polynucleotide sequences are detected in the same sample. In some embodiments, the plurality of target polynucleotides comprises more than two target polynucleotides and less than 10,000 target polynucleotides.

In a further aspect, the disclosure relates a method of detecting methylation of cytosine residues in a target polynucleotide in a sample comprising DNA. The method may include digesting the DNA in the sample with a methyl-insensitive restriction enzyme to create a target polynucleotide comprising a plurality of cytosine residues, wherein one or more of the cytosine residues are methylated; ligating one end of the target polynucleotide to a Unique Molecular Identifier (UMI) polynucleotide and the other end of the target polynucleotide to a protective polynucleotide to form a ligated polynucleotide, wherein a portion of the UMI polynucleotide comprises a first randomly generated UMI polynucleotide sequence, wherein all of the cytosine residues of the UMI polynucleotide are unmethylated, and wherein the protective polynucleotide includes an exonuclease resistant moiety; contacting the sample with one or more exonucleases adapted to digest any polynucleotides in the sample that do not include the exonuclease resistant moiety; forming a converted polynucleotide by chemically and/or enzymatically converting each methylated cytosine in the first ligated polynucleotide to dihydrouracil; amplifying the converted polynucleotide to generate a plurality of amplicon polynucleotides; sequencing the plurality of amplicon polynucleotides to generate a plurality of amplicon sequence reads, wherein each of the amplicon sequence reads: corresponds to the polynucleotide sequence of one of the plurality of amplicon polynucleotides; includes the randomly generated UMI polynucleotide sequence; and includes a thymine at each nucleotide position corresponding to the nucleotide position of a methylated cytosine in the target polynucleotide and a cytosine at each nucleotide position corresponding to an unmethylated cytosine in the target polynucleotide unless a conversion error during the conversion step, an amplification error during the amplification step or a sequencing error during the sequencing step causes the amplicon sequence read to include a nucleotide other than thymine at a nucleotide position corresponding to the nucleotide position of a methylated cytosine in the target polynucleotide or to include a nucleotide other than cytosine at a nucleotide position corresponding to the nucleotide position of an unmethylated cytosine in the target polynucleotide; and aligning the plurality of amplicon sequencing reads with a target polynucleotide reference sequence; if the sequencing step generated at least five amplicon sequence reads, then generating a consensus polynucleotide sequence corresponding to the polynucleotide sequence of the target polynucleotide, wherein generating the consensus polynucleotide sequence comprises: identifying each 5'-C-G-3' nucleotide pair in the target polynucleotide reference sequence; comparing each amplicon sequencing read to the target polynucleotide reference sequence to determine the identity of each nucleotide in each amplicon sequencing read aligned with each cytosine in each 5'-C-G-3' nucleotide pair in the target polynucleotide reference sequence; if 50% or more of the amplicon sequencing reads include a thymine at a position aligned with a cytosine in a 5'-C-G-3' nucleotide pair in the target polynucleotide reference sequence, then assigning a methylated cytosine to the corresponding position in the consensus polynucleotide sequence; and if less than 50% of the amplicon sequencing reads include a thymine at a position aligned with a cytosine in a 5'-C-G-3' nucleotide pair in the target polynucleotide reference sequence, then assigning an unmethylated cytosine to the corresponding position in the consensus polynucleotide sequence; and wherein if at least 50% of the 5'-C-G-3' nucleotide pairs in the consensus polynucleotide sequence have been assigned a methylated cytosine, then designating the target polynucleotide as methylated, and wherein if less than 50% of the 5'-C-G-3' nucleotide pairs in the consensus polynucleotide sequence have been assigned a methylated cytosine, then designating the target polynucleotide as unmethylated.

In some embodiments, the method further includes calculating the fraction of methylated target polynucleotides in the sample by dividing the number of methylated target polynucleotides by the total number of target polynucleotides detected in the sample. In some embodiments, the exonuclease resistant modification comprises a phosphorothioate bond and/or a 3-carbon spacer. In some embodiments, ligating the target polynucleotide to the UMI polynucleotide comprises annealing a first patch polynucleotide to both the target polynucleotide and the UMI polynucleotide, and wherein ligating the target polynucleotide to the protective polynucleotide comprises annealing a second patch polynucleotide to both the target polynucleotide and the protective polynucleotide. In some embodiments, TET enzymes and borane are used to convert the ligated polynucleotide to the converted polynucleotide. In some embodiments, the converted polynucleotide is amplified using the polymerase chain reaction (PCR). In some embodiments, the target polynucleotide is from a region of a genome known to be methylated in a specific cell type. In some embodiments, the specific cell type is a specific type of cancer cell. In some embodiments, the specific type of cancer cell is a cancer cell selected from the group consisting of breast cancer, ovarian cancer, lung cancer, pancreatic cancer, colorectal cancer, prostate cancer, uterine cancer, bladder cancer, and liver cancer. In some embodiments, generating the consensus polynucleotide sequence comprises assigning a methylated cytosine to the corresponding position in the consensus polynucleotide sequence if 90% or more of the amplicon sequencing reads include a thymine at a position aligned with a cytosine in a 5'-C-G-3' nucleotide pair in the target polynucleotide reference sequence, and assigning an unmethylated cytosine to the corresponding position in the consensus polynucleotide sequence if less than 90% of the amplicon sequencing reads include a thymine at a position aligned with a cytosine in a 5'-C-G-3' nucleotide pair in the target polynucleotide reference sequence. In some embodiments, the target polynucleotide is designated as methylated if at least 75% of the 5'-C-G-3' nucleotide pairs in the consensus polynucleotide sequence have been assigned a methylated cytosine, and the target polynucleotide is designated as unmethylated if less than 75% of the 5'-C-G-3' nucleotide pairs in the consensus polynucleotide sequence have been assigned a methylated cytosine. In some embodiments, the consensus polynucleotide comprises a plurality of 5'-C-G-3' nucleotide pairs. In some embodiments, methylation of cytosine residues in a plurality of target polynucleotide sequences are detected in the same sample. In some embodiments, the plurality of target polynucleotides comprises more than two target polynucleotides and less than 10,000 target polynucleotides.

Another aspect of the disclosure provides a method of diagnosing a patient with cancer. The method may include detecting methylation of cytosine residues in a target polynucleotide in a sample from the patient, wherein the methylation of cytosine residues is detected according to a method as detailed herein, and wherein the patient is diagnosed with cancer when methylation of cytosine residues in a target polynucleotide in the sample from the patient is detected. In some embodiments, the cancer is selected from the group consisting of breast cancer, ovarian cancer, lung cancer, pancreatic cancer, colorectal cancer, prostate cancer, uterine cancer, bladder cancer, and liver cancer. In some embodiments, method further includes treating the patient diagnosed with cancer by administering chemotherapy, radiation, immunotherapy, surgical resection, or a combination thereof.

This disclosure provides for other aspects and embodiments that will be apparent in light of the following detailed description and accompanying figures.

### BRIEF DESCRIPTION OF THE DRAWINGS

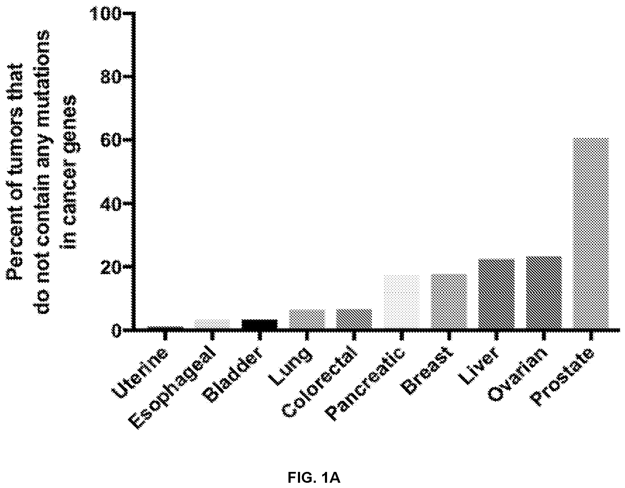

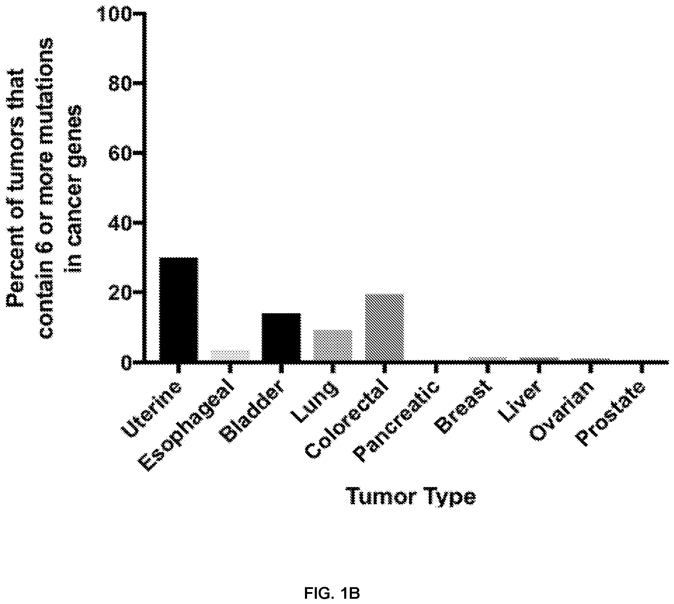

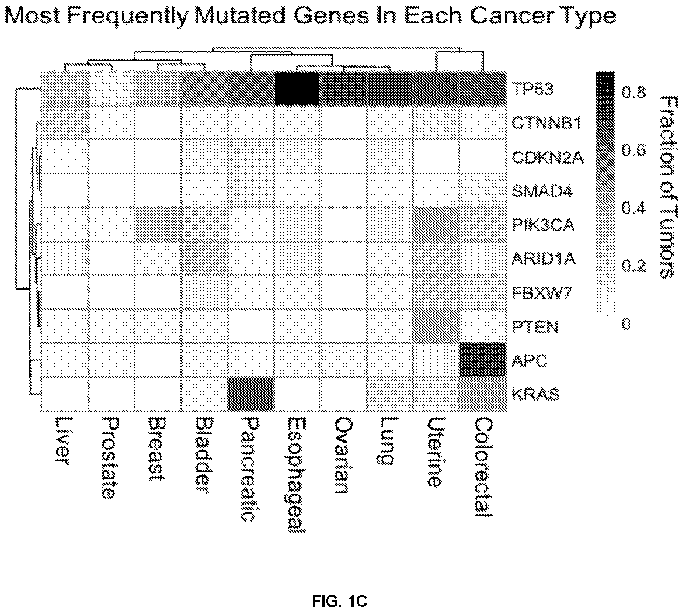

**FIG. 1A** is a graph showing that many tumors do not contain mutations in the 74 cancer genes covered by mutation-based ctDNA assays. FIG. 1B is a graph showing that only a small fraction of tumors have 6 or more mutations to enable sensitive detection of ctDNA in Stage I disease. FIG. 1C is a diagram demonstrating that the most common mutations in each cancer are found in other cancer types.

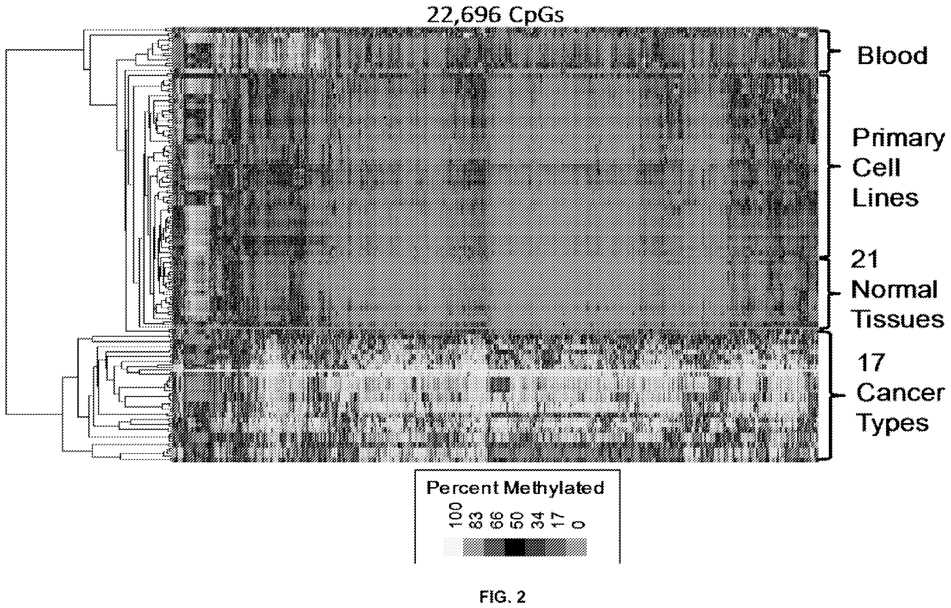

**FIG. 2** is a diagram showing the thousands of loci that are hypermethylated across cancer types and unmethylated in normal tissues.

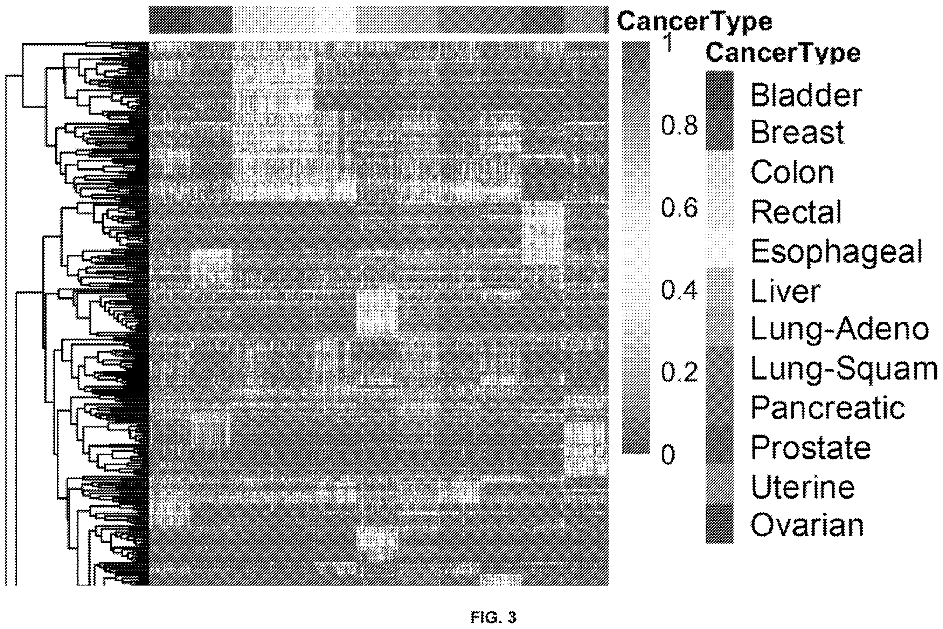

**FIG. 3** is a heatmap of genomic loci that exhibit cancer-type specific hypermethylation in TCGA data.

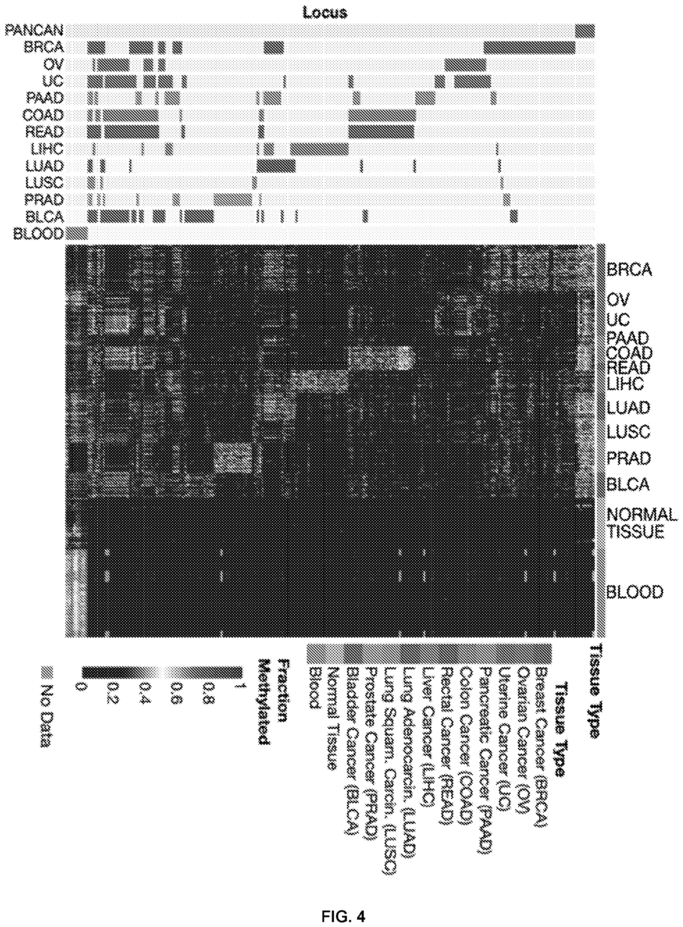

**FIG. 4** is a heatmap of 219 CG positions selected for the panel. There were 210 CGs selected because they had higher levels of methylation in breast cancer, ovarian cancer, uterine cancer, pancreatic cancer, colon cancer, rectal cancer, liver cancer, lung cancer, prostate cancer, and/or bladder cancer compared to normal tissue and blood from individuals without cancer. Additionally, 9 CGs were included in the panel because they are methylated in blood cells and unmethylated in tumor and normal tissues.

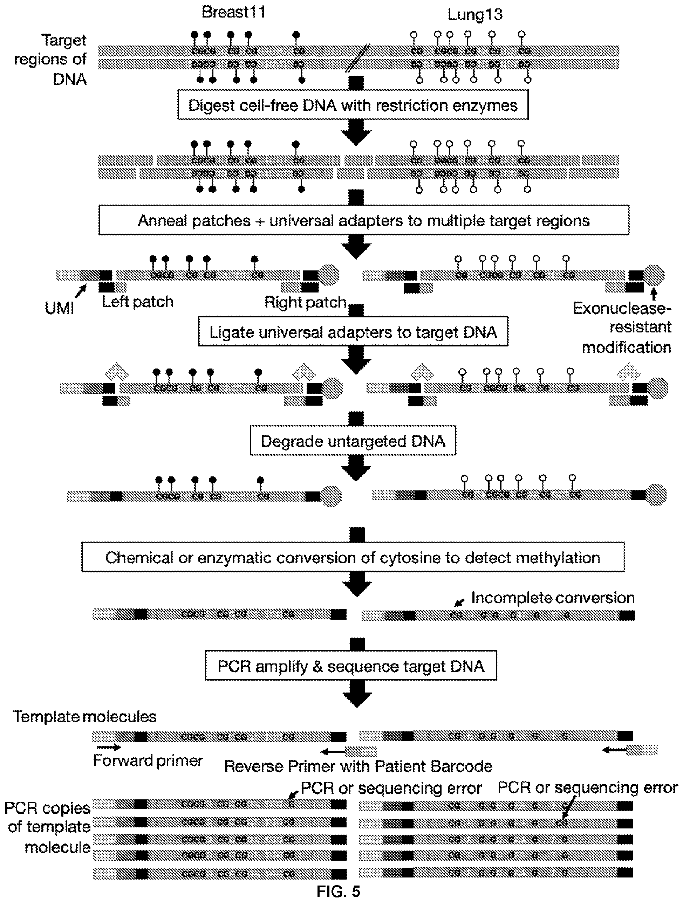

**FIG. 5** is a schematic diagram of the Methyl Patch PCR assay.

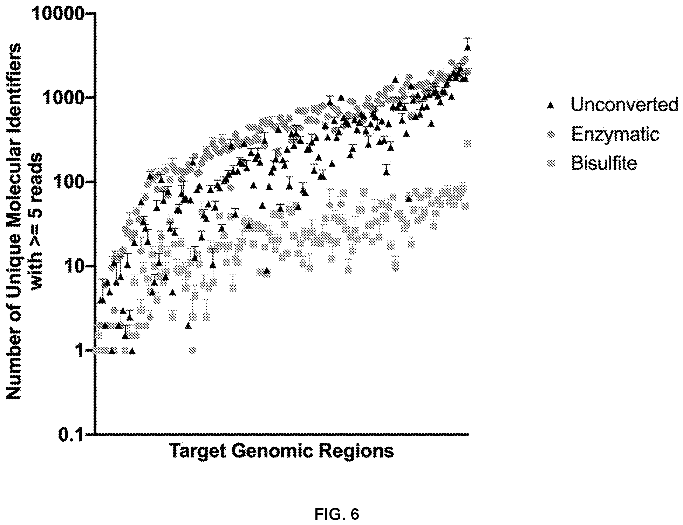

**FIG. 6** is a graph comparing the number of unique molecular identifiers sequenced when Methyl Patch PCR is performed with no cytosine conversion (unconverted), enzymatic cytosine conversion, and sodium bisulfite conversion. Mean and standard error of the mean (SEM) across 2 replicate experiments are depicted.

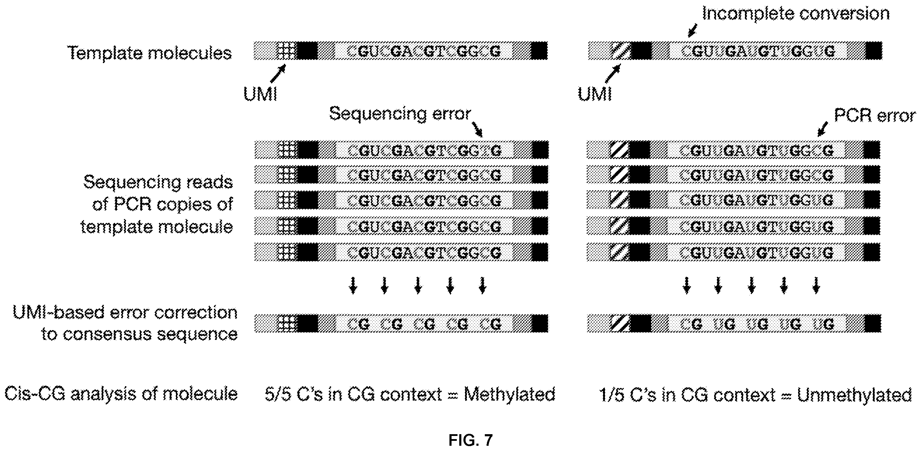

**FIG. 7** is a diagram of bioinformatics analysis to detect low levels of methylated DNA.

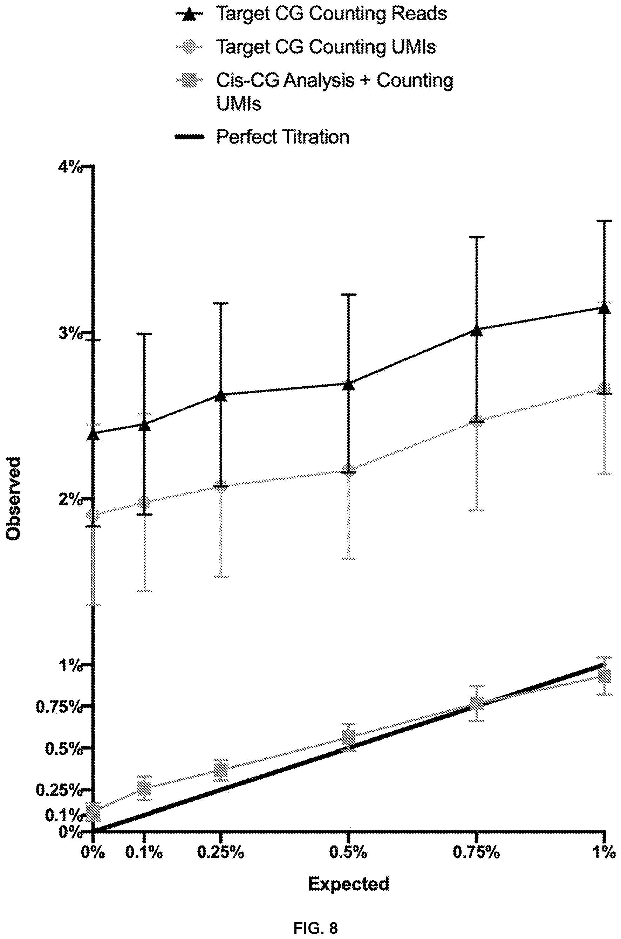

**FIG. 8** is a graph showing analysis of Methyl Patch PCR performed on a titration of low levels of methylated DNA into DNA isolated from healthy donor blood plasma. The graph shows the percentage of methylated reads at the target CG (triangle), percentage of methylated unique molecular identifiers at the target CG (circle), and cis-CG analysis of methylation at multiple CGs within the molecule (square). Means and 95% confidence intervals (whiskers) across target genomic regions are depicted.

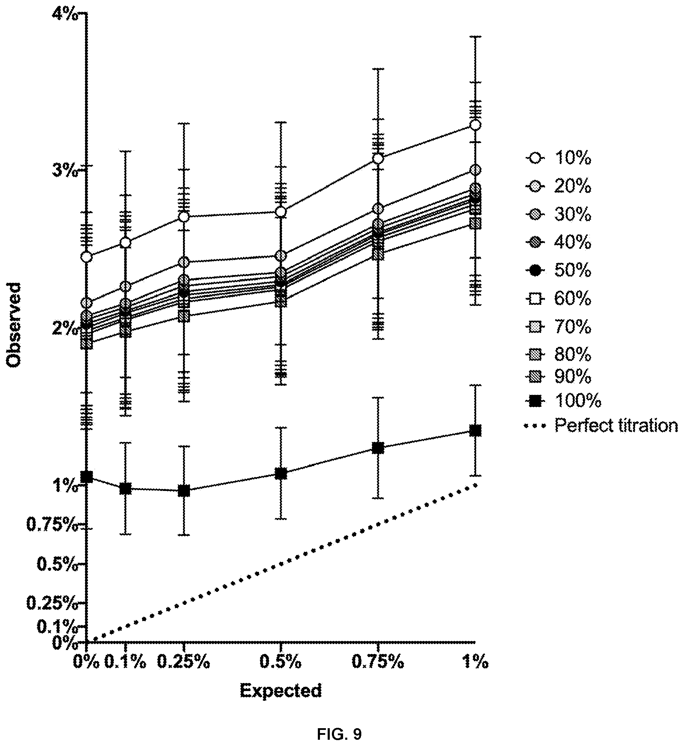

**FIG. 9** is a graph showing the evaluation of consensus call thresholds using samples containing a titration of low levels of methylated DNA into DNA isolated from healthy donor blood plasma. The graph shows the percentage of unique molecular identifiers that appeared methylated at the target CG calculated using different consensus call thresholds (10-100%). Means (dots) and 95% confidence intervals (whiskers) across target genomic regions are depicted.

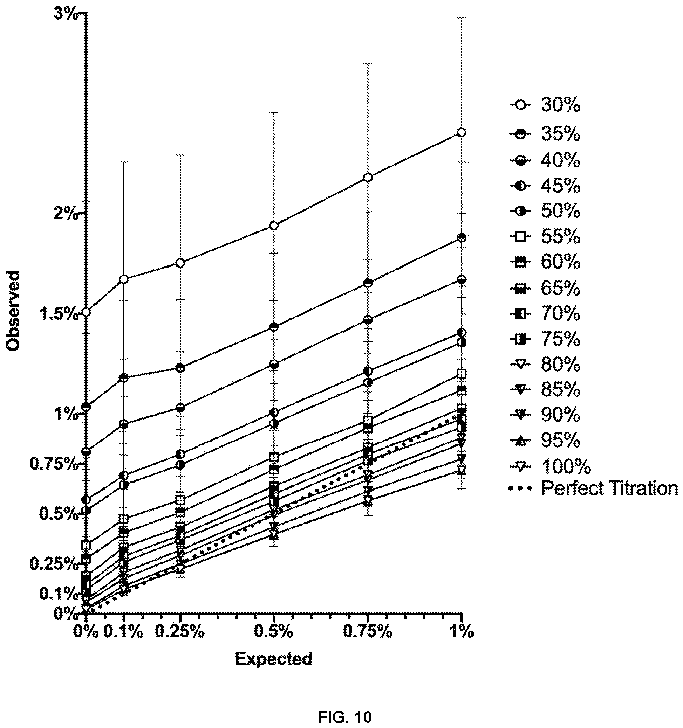

**FIG. 10** is a graph showing evaluation of cis-CG thresholds using samples containing a titration of low levels of methylated DNA into unmethylated DNA. The graph shows the percentage of unique molecular identifiers that appeared methylated at the target CG calculated using different cis-CG thresholds (30-100%). Means (dots) and 95% confidence intervals (whiskers) across target genomic regions are depicted.

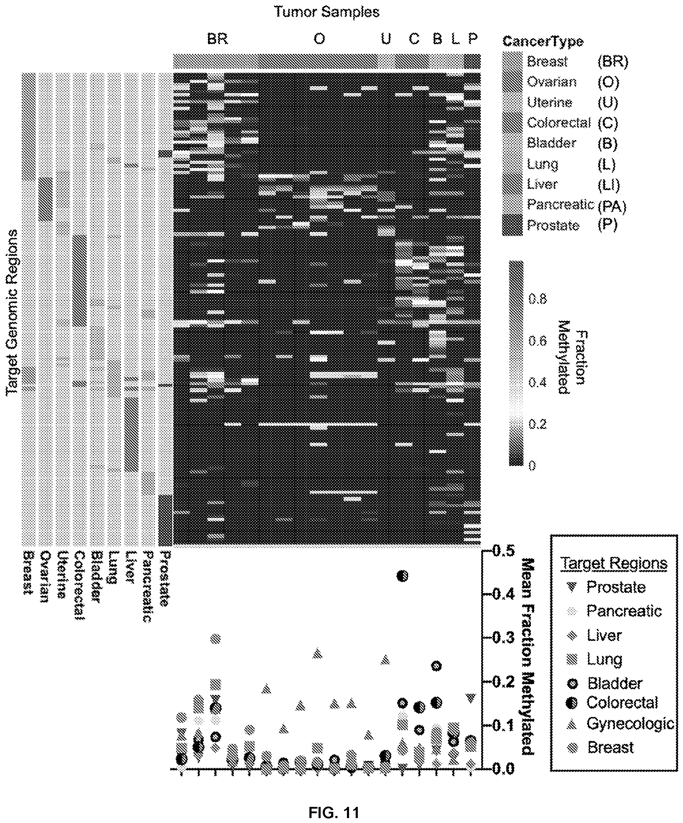

**FIG. 11** is a diagram showing the detection of methylated DNA in breast cancer, ovarian cancer, uterine cancer, colorectal cancer, bladder cancer, lung cancer, and prostate cancer. The heatmap depicts the fraction of molecules that appeared methylated at each target genomic region. Target genomic regions were grouped into sets that are named for the cancer type they are expected to be methylated in based on previous studies. The dots below each sample indicate the mean fraction of methylated molecules across the target regions for each cancer type set. The cancer type set with the highest mean fraction methylated in each sample corresponds to the cancer type of each sample.

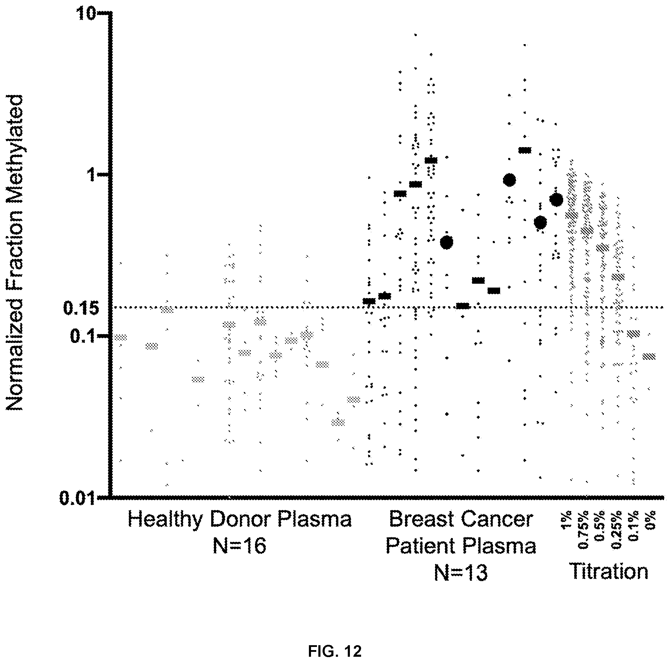

**FIG. 12** is a graph showing the normalized fraction of methylated molecules for target regions detected above background in healthy donor blood plasma (light grey), blood plasma collected from breast cancer patients in the interval between diagnosis and surgery (black), and in a titration of 1%, 0.75%, 0.5%, 0.25%, 0.1 %, and 0% methylated DNA into healthy donor plasma (dark grey). Each dot represents a target region. Mean values for each sample are indicated by a rectangle or circle. The threshold that distinguishes the mean values in all healthy donor samples from the mean values in all breast cancer samples is indicated by a dashed line at 0.15. Stage I breast cancer patients are indicated by a circle at the mean value.

### DETAILED DESCRIPTION

Described herein are compositions and methods for the detection and quantification of low levels of methylated DNA. The compositions and methods detailed herein involve multiplexed targeted sequencing and unique bioinformatics analysis to enable accurate detection of low levels (<1%) of methylated DNA in biological samples. The method utilizes our Patch PCR technology (U.S. Patent Nos. 8,936,912 B2 and 9,909,170 B2) to perform multiplexed capture of polynucleotides from specific regions of the genome. The method used to capture specific targeted polynucleotide regions of the genome enables the interrogation of molecules containing multiple sites of DNA methylation (5'-C-G-3' dinucleotides or CGs). This method was modified to include unique molecular identifiers (UMIs) that are ligated to the captured fragments. This method was also modified to treat the DNA with the chemicals and enzymes to convert the cytosine bases to distinguish methylated from unmethylated cytosine. The captured DNA may then be amplified and sequenced. Bioinformatic analysis of the sequencing data involves using UMIs to correct errors generated in PCR amplification and sequencing to determine a consensus sequence for the target polynucleotide molecule. Additionally, methylation across the consensus polynucleotide sequence for each molecule is analyzed to distinguish false-positive conversion errors from molecules that were methylated at multiple cytosines within the target fragment. In this way, the bioinformatics analysis of the sequencing data enables detection and quantification of molecules that are methylated at multiple sites, and distinguishes them from molecules that have false-positive conversion errors or sequencing errors at a subset of CGs within the molecule. This approach enables accurate detection of methylated DNA that is present at frequencies less the 1% in the sample, which is currently not feasible with other approaches. The multiplexed nature of this approach allows multiple target polynucleotides from different regions of the genome to be queried for low levels of methylated DNA simultaneously in the same capture and sequencing reaction. This feature provides more robust identification of methylation patterns specific to tumors, organs, cells, or species in mixed samples.

A non-invasive blood test for early diagnosis of cancer would significantly reduce cancer mortality and health care costs. The discovery that cells shed DNA into the bloodstream (cfDNA), and that cancer-specific mutations can be detected by ultra-deep sequencing of cell free DNA from blood plasma has revolutionized the field of cancer diagnostics. This strategy has been very successful for detecting some types of late-stage cancers, such as lung and melanoma, which produce large amounts of cell-free DNA (cfDNA) and frequently have hotspot mutations in cancer-associated genes, because these features provide a robust signal for detecting cfDNA shed from tumors, which is known as circulating tumor DNA (ctDNA).

Recently several groups have attempted to use mutation based ctDNA assays for detection of early stage tumors, however it has become clear that there are 3 main challenges that limit the effectiveness of ctDNA mutation assays for early detection of common cancers:

1. Many patient tumors lack mutations in 'cancer genes'. We analyzed tumor mutation data from The Cancer Genome Atlas (TCGA) and found that many tumors do not contain point mutations in 73 cancer genes covered by Guardant Health's field-leading ctDNA assay (Guardant 360^{®})(Lanman, R.B., et al. PLoS One 2015, 10, e0140712) (**FIG. 1A**)**.** This analysis indicated that even if a mutation-based ctDNA test had perfect analytical sensitivity, it would be unable detect cancer in a large number of patients with common cancers, including 18% (189/1066) of breast cancers and 61% (300/494) of prostate cancers, which are the most common cancers diagnosed in the U.S. in women and men, respectively.

2. Early stage tumors produce lower amounts of cell-free DNA. In three recent studies that evaluated ctDNA mutation assays for cancer detection, the frequency of ctDNA detection in Stage 1 patients was approximately 50%. This suggests that tumor-specific mutations were present at a concentration of 0.5 molecules per blood specimen. One way to increase the probability of observing rare molecules is to increase the amount of blood collected from each patient, which is often prohibited by Institutional Review Boards to protect patient safety. Alternatively, measuring multiple mutations increases the probability of observing rare ctDNA. If you could detect 6 independent mutations in a patient, the probability of missing all 6 mutations due to undersampling is low, and 98% (1-0.5^6) of Stage I patients would have detectable ctDNA. We analyzed tumor mutation data from The Cancer Genome Atlas (TCGA) and found that only a small fraction of tumors had 6 or more mutations in cancer genes (FIG. 1B). This result indicates that mutation-based assays are unlikely to be sensitive enough for detection of rare ctDNA molecules in early stage disease.

3. Mutations are not unique to specific cancers. An additional drawback of using ctDNA mutation assays for early diagnosis is that many different tumor types contain the same mutations. The genes that are most frequently mutated in one cancer type (>20% of tumors) are also frequently mutated in other cancer types (FIG. 1C). If a ctDNA test detected a TP53 mutation during routine screening, it would indicate that the individual had cancer, but because TP53 mutations occur in all tumor types, it does not predict the patient's tumor type, tumor location, or tissue of origin. This would lead to expensive follow-up imaging of multiple organs to identify the malignancy and determine which oncologist should manage the patient's care.

These three inherent challenges limit the effectiveness of ctDNA mutation assays for early detection of common cancers. Therefore, a different strategy is needed if ctDNA tests are to be used for screening.

An alternative strategy for detecting ctDNA is to identify cancer-specific DNA methylation. Aberrant DNA methylation occurs early in tumor development at hundreds of specific genomic loci in each tumor. Many cancer genomics research groups have demonstrated that hundreds of loci exhibit tumor-specific methylation across cancer types (FIG. 2). This aspect of DNA methylation directly address challenge #1 described above. While some patients' tumors do not contain mutations in cancer genes, every tumor will have cancer-specific DNA methylation at the commonly methylated loci. Measuring DNA methylation may enable detection of ctDNA in a larger portion of patients with cancer.

Additionally, an individual patient's tumor may contain many more methylation defects than mutations. This feature of DNA methylation directly addresses challenge #2 described above. While it is unlikely that a patient's tumor will contain 6 point mutations in cancer genes, the presence of 6 cancer-specific methylation events in a single patient is common, and expected, because hundreds of loci are concurrently hypermethylated in each tumor. A ctDNA test designed to detect cancer-specific hypermethylation at many loci would increase the probability of observing rare ctDNA molecules in the blood specimen. This would enable robust and sensitive detection of early stage tumors that produce lower amounts of cell-free DNA.

Finally, different cancer types have distinct methylation profiles **(****FIG. 3****)(**Yang, X., et al. Comparative pan-cancer DNA methylation analysis reveals cancer common and specific patterns. Brief Bioinform, 2016; Moran, S., et al. Lancet Oncol. 2016, 17, 1386-1395; Kang, S., et al. Genome Biol. 2017, 18, 53). This directly addresses challenge #3 described above. While most mutations are not unique to specific cancer types, if a colon cancer-specific DNA methylation profile was detected in the blood, it would indicate a specific diagnosis of colon cancer. This means each cancer type can be diagnosed based on which loci are methylated, leading to a more definitive diagnosis and more straightforward referral to the appropriate oncology specialist. The robust prediction of the tumor type and location is a critical capability if ctDNA tests are to be used for screening the general population.

For all of these reasons, detection of methylated ctDNA promises to provide a more robust and specific signal for ctDNA detection in early stage disease.

The ability to detect ctDNA based on cancer-specific methylation has been hampered by our ability to detect DNA methylation by sequencing. DNA polymerases do not copy the methylation state of the template DNA molecules. The current gold standard approach is bisulfite sequencing where the DNA is treated with sodium bisulfite to convert unmethylated cytosine to uracil, leaving methylated cytosine intact. During subsequent PCR amplification, uracil is copied as thymine and methylated cytosine is copied as cytosine. Unfortunately, this chemical conversion is necessarily a harsh treatment, which causes extensive DNA damage in the form of nicks, fragmentation, and abasic sites (Ehrich, M., et al., Nucleic Acids Res. 2007, 35, e29; Grunau, C., et al. Nucleic Acids Res. 2001, 29, E65-E65). The damage from bisulfite treatment becomes particularly problematic for accurate detection of rare molecules, such as ctDNA. Furthermore, the best reported bisulfite conversion efficiency in high quality control DNA is 99.5%, which means that 0.5% of unmethylated cytosines will not be converted and will appear as false-positive methylation events. This false-positive rate makes it difficult to distinguish rare methylated ctDNA molecules from molecules with incomplete bisulfite conversion.

As detailed in the Examples, methods were developed to detect and quantify circulating tumor DNA (ctDNA) present at low levels in cell-free DNA (cfDNA) isolated from blood plasma.

The compositions and methods detailed herein to detect and quantify low levels of methylated DNA may enable many important applications including, for example, detecting tumor DNA in body fluids for the diagnosis and prognosis of cancer; detecting fetal DNA in body fluids of pregnant women for diagnosis and prognosis of pregnancy; detecting DNA methylation in forensic samples that is indicative of age, gender, and ethnicity; detecting cell-type specific methylation in body fluids to diagnose organ disease or damage; detecting tumor-type specific methylation in body fluids to determine tumor origin in patients diagnosed with Cancer of Unknown Primary; quantifying tumor cell content in specimens and cultures derived from patients; and detecting species-specific and strain-specific DNA methylation in environmental samples to track organisms in the environment.

### 1. Definitions

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present invention. The materials, methods, and examples disclosed herein are illustrative only and not intended to be limiting.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and" and "the" include plural references unless the context clearly dictates otherwise. The present disclosure also contemplates other embodiments "comprising," "consisting of" and "consisting essentially of," the embodiments or elements presented herein, whether explicitly set forth or not.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

The term "about" as used herein as applied to one or more values of interest, refers to a value that is similar to a stated reference value. In certain aspects, the term "about" refers to a range of values that fall within 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

"Cancer" refers to a neoplasm or tumor resulting from abnormal and uncontrolled growth of cells. Cancer may also be referred to as a cellular-proliferative disease. Cancer may include different histological types, cell types, and different stages of cancer, such as, for example, primary tumor or metastatic growth. Cancer may include, for example, breast cancer, cholangiocellular carcinoma, colorectal cancer, endometriosis, esophageal cancer, gastric cancer, diffused type gastric cancer, pancreatic cancer, renal carcinoma, soft tissue tumor, testicular cancer, cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hanlartoma, inesothelioma, non-small cell lung cancer (NSCLC), small cell lung cancer (SCLC); Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor [nephroblastoma], lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis defornians), meninges (meningioma, meningiosarcoma, gliomatosis), brain (astrocytoma, medulloblastoma, glioma, ependymoma, germinoma [pinealoma], glioblastoma, glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian cancer, ovarian carcinoma [serous cystadenocarcinoma, mucinous cystadenocarcinoma, unclassified carcinoma], granulosa-thecal cell tumors, SertoliLeydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); Hematologic: blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma], CML; Skin: melanoma, malignant melanoma, basal cell carcinoma, squamous cell carcinoma, Karposi's sarcoma, moles, dysplastic nevi, lipoma, angioma, dermatofibroma, keloids, psoriasis; and Adrenal glands: neuroblastoma. In some embodiments, the cancer comprises non-small cell lung cancer (NSCLC). In some embodiments, the cancer comprises at least one of breast cancer, ovarian cancer, lung cancer, pancreatic cancer, colorectal cancer, prostate cancer, uterine cancer, bladder cancer, and liver cancer.

The terms "control," "reference level," and "reference" are used herein interchangeably. The reference level may be a predetermined value or range, which is employed as a benchmark against which to assess the measured result. "Control group" as used herein refers to a group of control subjects. The predetermined level may be a cutoff value from a control group. The predetermined level may be an average from a control group. Cutoff values (or predetermined cutoff values) may be determined by Adaptive Index Model (AIM) methodology. Cutoff values (or predetermined cutoff values) may be determined by a receiver operating curve (ROC) analysis from biological samples of the patient group. ROC analysis, as generally known in the biological arts, is a determination of the ability of a test to discriminate one condition from another, e.g., to determine the performance of each marker in identifying a patient having CRC. A description of ROC analysis is provided in P.J. Heagerty et al. (Biometrics 2000, 56, 337-44).

Alternatively, cutoff values may be determined by a quartile analysis of biological samples of a patient group. For example, a cutoff value may be determined by selecting a value that corresponds to any value in the 25th-75th percentile range, preferably a value that corresponds to the 25th percentile, the 50th percentile or the 75th percentile, and more preferably the 75th percentile. Such statistical analyses may be performed using any method known in the art and can be implemented through any number of commercially available software packages (e.g., from Analyse-it Software Ltd., Leeds, UK; StataCorp LP, College Station, TX; SAS Institute Inc., Cary, NC.). The healthy or normal levels or ranges for a target or for a protein activity or for a gene expression level may be defined in accordance with standard practice. A control may be a subject, or a sample therefrom, whose disease state is known. The subject, or sample therefrom, may be at any stage of disease. The subject, or sample therefrom, may be healthy, diseased, diseased prior to treatment, diseased during treatment, or diseased after treatment, or a combination thereof.

"Polynucleotide" as used herein can be single stranded or double stranded, or can contain portions of both double stranded and single stranded sequence. The polynucleotide can be nucleic acid, natural or synthetic, DNA, genomic DNA, cDNA, RNA, or a hybrid, where the polynucleotide can contain combinations of deoxyribo- and ribonucleotides, and combinations of bases including uracil, adenine, thymine, cytosine, guanine, inosine, xanthine hypoxanthine, isocytosine, and isoguanine. Polynucleotides can be obtained by chemical synthesis methods or by recombinant methods.

Polynucleotides are said to have "5' ends" and "3' ends" because mononucleotides are reacted to make oligonucleotides in a manner such that the 5' phosphate of one mononucleotide pentose ring is attached to the 3' oxygen of its neighbor in one direction via a phosphodiester linkage. Therefore, an end of an oligonucleotide is referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a polynucleotide sequence, even if internal to a larger oligonucleotide, also may be said to have 5' and 3' ends. In either a linear or circular polynucleotide, discrete elements are referred to as being "upstream" or 5' of the "downstream" or 3' elements. This terminology reflects the fact that transcription proceeds in a 5' to 3' fashion along the polynucleotide strand. The promoter and enhancer elements which direct transcription of a linked gene are generally located 5' or upstream of the coding region. However, enhancer elements can exert their effect even when located 3' of the promoter element and the coding region. Transcription termination and polyadenylation signals are located 3' or downstream of the coding region.

A "peptide" or "polypeptide" is a linked sequence of two or more amino acids linked by peptide bonds. The polypeptide can be natural, synthetic, or a modification or combination of natural and synthetic. Peptides and polypeptides include proteins such as binding proteins, receptors, and antibodies. The terms "polypeptide", "protein," and "peptide" are used interchangeably herein. "Primary structure" refers to the amino acid sequence of a particular peptide. "Secondary structure" refers to locally ordered, three dimensional structures within a polypeptide. These structures are commonly known as domains, e.g., enzymatic domains, extracellular domains, transmembrane domains, pore domains, and cytoplasmic tail domains. "Domains" are portions of a polypeptide that form a compact unit of the polypeptide and are typically 15 to 350 amino acids long. Exemplary domains include domains with enzymatic activity or ligand binding activity. Typical domains are made up of sections of lesser organization such as stretches of beta-sheet and alpha-helices. "Tertiary structure" refers to the complete three dimensional structure of a polypeptide monomer. "Quaternary structure" refers to the three dimensional structure formed by the noncovalent association of independent tertiary units. A "motif' is a portion of a polypeptide sequence and includes at least two amino acids. A motif may be 2 to 20, 2 to 15, or 2 to 10 amino acids in length. In some embodiments, a motif includes 3, 4, 5, 6, or 7 sequential amino acids. A domain may be comprised of a series of the same type of motif.

As used herein, the term "restriction endonuclease" or "restriction enzyme" refers to a member or members of a classification of catalytic molecules that bind a cognate sequence of a polynucleotide and cleave the polynucleotide at a precise location within that sequence. Restriction endonuclease may be bacterial enzymes. Restriction endonuclease may cut double-stranded DNA at or near a specific nucleotide sequence.

As used herein, "recognition site" or "restriction site" refers to a sequence of specific bases or nucleotides that is recognized by a restriction enzyme if the sequence is present in double-stranded DNA; or, if the sequence is present in single-stranded RNA, the sequence of specific bases or nucleotides that would be recognized by a restriction enzyme if the RNA was reverse transcribed into cDNA and the cDNA employed as a template with a DNA polymerase to generate a double-stranded DNA; or, if the sequence is present in single-stranded DNA, the sequence of specific bases or nucleotides that would be recognized by a restriction enzyme if the single-stranded DNA was employed as a template with a DNA polymerase to generate a double-stranded DNA; or, if the sequence is present in double-stranded RNA, the sequence of specific bases or nucleotides that would be recognized by a restriction enzyme if either strand of RNA was reverse transcribed into cDNA and the cDNA employed as a template with a DNA polymerase to generate a double-stranded DNA. The term "unique restriction enzyme site" or "unique recognition site" indicates that the recognition sequence for a given restriction enzyme appears once within a polynucleotide.

"Sample" or "test sample" as used herein can mean any sample in which the presence and/or level of a target or gene is to be detected or determined. Samples may include liquids, solutions, emulsions, or suspensions. Samples may include a medical sample. Samples may include any biological fluid or tissue, such as blood, whole blood, fractions of blood such as plasma and serum, muscle, interstitial fluid, sweat, saliva, urine, tears, synovial fluid, bone marrow, cerebrospinal fluid, nasal secretions, sputum, amniotic fluid, bronchoalveolar lavage fluid, gastric lavage, emesis, fecal matter, lung tissue, peripheral blood mononuclear cells, total white blood cells, lymph node cells, spleen cells, tonsil cells, cancer cells, tumor cells, bile, digestive fluid, skin, or combinations thereof. In some embodiments, the sample comprises an aliquot. In other embodiments, the sample comprises a biological fluid. Samples can be obtained by any means known in the art. The sample can be used directly as obtained from a patient or can be pre-treated, such as by filtration, distillation, extraction, concentration, centrifugation, inactivation of interfering components, addition of reagents, and the like, to modify the character of the sample in some manner as discussed herein or otherwise as is known in the art.

"Subject" as used herein can mean a mammal that wants or is in need of the herein described assays or methods. The subject may be a patient. The subject may be a human or a non-human animal. The subject may be a mammal. The mammal may be a primate or a non-primate. The mammal can be a primate such as a human; a non-primate such as, for example, dog, cat, horse, cow, pig, mouse, rat, camel, llama, goat, rabbit, sheep, hamster, and guinea pig; or non-human primate such as, for example, monkey, chimpanzee, gorilla, orangutan, and gibbon. The subject may be of any age or stage of development, such as, for example, an adult, an adolescent, or an infant. The subject may be male or female. In some embodiments, the subject has a specific genetic marker.

"Substantially identical" can mean that a first and second polynucleotide or amino acid sequence are at least 60%, 65%, 70%, 75%, 80%, 85%, 90%, 95%, 96%, 97%, 98%, or 99% over a region of 1, 2, 3, 4, 5, 6, 7, 8, 9, 10, 11, 12, 13, 14, 15, 16, 17, 18, 19, 20, 21, 22, 23, 24, 25, 30, 35, 40, 45, 50, 55, 60, 65, 70, 75, 80, 85, 90, 95, 100, 200, 300, 400, 500, 600, 700, 800, 900, 1000, 1100 amino acids.

"Variant" as used herein with respect to a polynucleotide means (i) a portion or fragment of a referenced nucleotide sequence; (ii) the complement of a referenced nucleotide sequence or portion thereof; (iii) a polynucleotide that is substantially identical to a referenced polynucleotide or the complement thereof; or (iv) a polynucleotide that hybridizes under stringent conditions to the referenced polynucleotide, complement thereof, or a sequence substantially identical thereto. A variant can be a polynucleotide sequence that is substantially identical over the full length of the full gene sequence or a fragment thereof. The polynucleotide sequence can be 80%, 81%, 82%, 83%, 84%, 85%, 86%, 87%, 88%, 89%, 90%, 91%, 92%, 93%, 94%, 95%, 96%, 97%, 98%, 99%, or 100% identical over the full length of the gene sequence or a fragment thereof.

A "variant" can further be defined as a peptide or polypeptide that differs in amino acid sequence by the insertion, deletion, or conservative substitution of amino acids, but retain at least one biological activity. Representative examples of "biological activity" include the ability to be bound by a specific antibody or polypeptide or to promote an immune response. Variant can mean a substantially identical sequence. Variant can mean a functional fragment thereof. Variant can also mean multiple copies of a polypeptide. The multiple copies can be in tandem or separated by a linker. Variant can also mean a polypeptide with an amino acid sequence that is substantially identical to a referenced polypeptide with an amino acid sequence that retains at least one biological activity. A conservative substitution of an amino acid, i.e., replacing an amino acid with a different amino acid of similar properties (e.g., hydrophilicity, degree and distribution of charged regions) is recognized in the art as typically involving a minor change. These minor changes can be identified, in part, by considering the hydropathic index of amino acids. See Kyte et al., J. Mol. Biol. 1982, 157, 105-132. The hydropathic index of an amino acid is based on a consideration of its hydrophobicity and charge. It is known in the art that amino acids of similar hydropathic indexes can be substituted and still retain protein function. In one aspect, amino acids having hydropathic indices of ±2 are substituted. The hydrophobicity of amino acids can also be used to reveal substitutions that would result in polypeptides retaining biological function. A consideration of the hydrophilicity of amino acids in the context of a polypeptide permits calculation of the greatest local average hydrophilicity of that polypeptide, a useful measure that has been reported to correlate well with antigenicity and immunogenicity, as discussed in U.S. Patent No. 4,554,101. Substitution of amino acids having similar hydrophilicity values can result in polypeptides retaining biological activity, for example immunogenicity, as is understood in the art. Substitutions can be performed with amino acids having hydrophilicity values within ±2 of each other. Both the hydrophobicity index and the hydrophilicity value of amino acids are influenced by the particular side chain of that amino acid. Consistent with that observation, amino acid substitutions that are compatible with biological function are understood to depend on the relative similarity of the amino acids, and particularly the side chains of those amino acids, as revealed by the hydrophobicity, hydrophilicity, charge, size, and other properties. A variant can be an amino acid sequence that is substantially identical over the full length of the amino acid sequence or fragment thereof. The amino acid sequence can be 80%, 81%, 82%, 83%, 84%, 85%, 86%, 87%, 88%, 89%, 90%, 91%, 92%, 93%, 94%, 95%, 96%, 97%, 98%, 99%, or 100% identical over the full length of the amino acid sequence or a fragment thereof.

"Variant" as used herein with respect to DNA methylation refers to polynucleotides that differ in the total number of nucleotides methylated, in the proportion of nucleotides methylated, and/or in the position of methylated or unmethylated nucleotide(s).

This disclosure provides methods of detecting methylation of cytosine residues in a target polynucleotide in a sample comprising DNA.

### 2. Methods of detecting methylation of cytosine residues involving chemical or enzymatic conversion of unmethylated cytosines to uracil

Provided herein are methods of detecting methylation of cytosine residues. In some embodiments, the methods involve chemical or enzymatic conversion of unmethylated cytosines to uracil. In some embodiments, the methodsinclude digesting the DNA in the sample with a methyl-insensitive restriction enzyme to create a target polynucleotide comprising a plurality of cytosine residues, wherein one or more of the cytosine residues are methylated; ligating one end of the target polynucleotide to a Unique Molecular Identifier (UMI) polynucleotide and the other end of the target polynucleotide to a protective polynucleotide to form a ligated polynucleotide, wherein a portion of the UMI polynucleotide comprises a first randomly generated UMI polynucleotide sequence, wherein all of the cytosine residues of the UMI polynucleotide are methylated, and wherein the protective polynucleotide includes an exonuclease resistant moiety; contacting the sample with one or more exonucleases adapted to digest any polynucleotides in the sample that do not include the exonuclease resistant moiety; forming a converted polynucleotide by chemically and/or enzymatically converting each unmethylated cytosine in the ligated polynucleotide to uracil; amplifying the converted polynucleotide to generate a plurality of amplicon polynucleotides; sequencing the plurality of amplicon polynucleotides to generate a plurality of amplicon sequence reads, wherein each of the amplicon sequence reads: corresponds to the polynucleotide sequence of one of the plurality of amplicon polynucleotides; includes the randomly generated UMI polynucleotide sequence; and includes a thymine at each nucleotide position corresponding to the nucleotide position of an unmethylated cytosine in the target polynucleotide and a cytosine at each nucleotide position corresponding to a methylated cytosine in the target polynucleotide unless a conversion error during the conversion step, an amplification error during the amplification step or a sequencing error during the sequencing step causes the amplicon sequence read to include a nucleotide other than thymine at a nucleotide position corresponding to the nucleotide position of an unmethylated cytosine in the target polynucleotide or to include a nucleotide other than cytosine at a nucleotide position corresponding to the nucleotide position of a methylated cytosine in the target polynucleotide; and aligning the plurality of amplicon sequencing reads with a target polynucleotide reference sequence. If the sequencing step generated at least five amplicon sequence reads, then the method may further include generating a consensus polynucleotide sequence corresponding to the polynucleotide sequence of the target polynucleotide, wherein generating the consensus polynucleotide sequence may include identifying each 5'-C-G-3' nucleotide pair in the target polynucleotide reference sequence; comparing each amplicon sequencing read to the target polynucleotide reference sequence to determine the identity of each nucleotide in each amplicon sequencing read aligned with each cytosine in each 5'-C-G-3' nucleotide pair in the target polynucleotide reference sequence. If 50% or more of the amplicon sequencing reads include a cytosine at a position aligned with a cytosine in a 5'-C-G-3' nucleotide pair in the target polynucleotide reference sequence, then the method may further include assigning a methylated cytosine to the corresponding position in the consensus polynucleotide sequence. If less than 50% of the amplicon sequencing reads include a cytosine at a position aligned with a cytosine in a 5'-C-G-3' nucleotide pair in the target polynucleotide reference sequence, then the method may further include assigning an unmethylated cytosine to the corresponding position in the consensus polynucleotide sequence. In some embodiments, if at least 50% of the 5'-C-G-3' nucleotide pairs in the consensus polynucleotide sequence have been assigned a methylated cytosine, then the method further includes designating the target polynucleotide as methylated. In some embodiments, if less than 50% of the 5'-C-G-3' nucleotide pairs in the consensus polynucleotide sequence have been assigned a methylated cytosine, then the method further includes designating the target polynucleotide as unmethylated.

The presence of one or more methylated target polynucleotides in the biological sample indicates the detection of methylated cytosine residues. In some embodiments, the presence of one or more methylated target polynucleotides in the biological sample indicates the detection of methylated DNA.

In some embodiments, the fraction of methylated target polynucleotides is calculated by dividing the number of methylated target polynucleotides by the total number of target polynucleotides detected in the biological sample. This fraction may be used to quantify low levels of methylated DNA in the biological sample.

In some embodiments, the exonuclease resistant modification may comprise a phosphorothioate bond and/or a 3-carbon spacer. The exonuclease resistant modification may comprise a plurality of phosphorothioate bonds and/or a plurality of 3-carbon spacers. For example, the exonuclease resistant modification may comprise two, three, four, five or six phosphorothioate bonds and/or two, three, four, five or six 3-carbon spacers. The modifications may be selected so as to inhibit or prevent digestion of the modification by one or more exonucleases, such as one or more 5' to 3' specific exonucleases or one or more 3' to 5' specific exonucleases, including but not limited to Exonuclease I, Exonuclease II, Exonuclease III, Exonuclease IV, Exonuclease V, Exonuclease VI, Exonuclease VII and Exonuclease VIII. For example, the protective polynucleotide may be ligated to the 5' end of the target polynucleotide such that the exonuclease resistant modification inhibits or prevents digestion of the target polynucleotide by one or more 5' to 3' exonucleases. Conversely, the protective polynucleotide may be ligated to the 3' end of the target polynucleotide such that the exonuclease resistant modification inhibits or prevents digestion of the target polynucleotide by one or more 3' to 5' exonucleases.

In some embodiments, ligating the target polynucleotide to the UMI polynucleotide may comprise annealing a first patch polynucleotide to both the target polynucleotide and the UMI polynucleotide. Ligation may be accomplished by methods well known in the art. For example, the first patch polynucleotide may include a first portion that is complementary to the target polynucleotide, and a second portion that is complementary to the UMI polynucleotide. After the first patch polynucleotide is annealed to the target polynucleotide and the UMI polynucleotide, the target polynucleotide and UMI polynucleotide can be ligated using DNA ligase. Similarly, ligating the target polynucleotide to the protective polynucleotide may comprise annealing a second patch polynucleotide to both the target polynucleotide and the protective polynucleotide. For example, the second patch polynucleotide may include a first portion that is complementary to the target polynucleotide, and a second portion that is complementary to the protective polynucleotide. After the second patch polynucleotide is annealed to the target polynucleotide and the protective polynucleotide, the target polynucleotide and protective polynucleotide can be ligated using DNA ligase.

In some embodiments, forming the converted polynucleotide may include enzymatically converting each unmethylated cytosine of the ligated polynucleotide to uracil. For example, TET2 and APOBEC may be used to enzymatically convert each cytosine to uracil. In other embodiments, the unmethylated cytosines may be chemically converted to uracil according to any method known in the art including, but not limited to, bisulfite conversion.

In some embodiments, the converted polynucleotide may be amplified using the polymerase chain reaction (PCR), although any method known in the art may be used to amplify the converted polynucleotide.

The target polynucleotide may be from a region of a genome known to be methylated in a specific cell type, such as a specific type of cancer cell. For example, the specific type of cancer cell may be selected from the group consisting of breast cancer, ovarian cancer, lung cancer, pancreatic cancer, colorectal cancer, prostate cancer, uterine cancer, bladder cancer, and liver cancer.

In some embodiments, generating the consensus polynucleotide sequence may comprise assigning a methylated cytosine to the corresponding position in the consensus polynucleotide sequence if 90% or more of the amplicon sequencing reads include a cytosine at a position aligned with a cytosine in a 5'-C-G-3' nucleotide pair in the target polynucleotide reference sequence, and assigning an unmethylated cytosine to the corresponding position in the consensus polynucleotide sequence if less than 90% of the amplicon sequencing reads include a cytosine at a position aligned with a cytosine in a 5'-C-G-3' nucleotide pair in the target polynucleotide reference sequence.

In some embodiments, the target polynucleotide may be designated as methylated if at least 75% of the 5'-C-G-3' nucleotide pairs in the consensus polynucleotide sequence have been assigned a methylated cytosine, and the target polynucleotide may be designated as unmethylated if less than 75% of the 5'-C-G-3' nucleotide pairs in the consensus polynucleotide sequence have been assigned a methylated cytosine.

It should be appreciated that the consensus polynucleotide may comprise a plurality of 5'-C-G-3' nucleotide pairs.

In some embodiments, methylation of cytosine residues in a plurality of target polynucleotide sequences may be detected in the same sample. For example, the plurality of target polynucleotides may comprise more than two target polynucleotides and less than 10,000 target polynucleotides, depending on the restriction enzymes used to digest the DNA in the sample.

Moreover, a plurality of copies of each target polynucleotide may be present in a sample, where each of the plurality of copies may be ligated to different UMI polynucleotides. As such, a plurality of ligated polynucleotides may be formed corresponding to each target polynucleotide, where each of these ligated polynucleotides include a different UMI polynucleotide.

### 3. Methods of detecting methylation of cytosine residues involving chemical or enzymatic conversion of methylated cytosines to dihydrouracil

In some embodiments, the methods of detecting methylation of cytosine residues involve chemical or enzymatic conversion of methylated cytosines to dihydrouracil. In some embodiments, the methods include digesting the DNA in the sample with a methyl-insensitive restriction enzyme to create a target polynucleotide comprising a plurality of cytosine residues, wherein one or more of the cytosine residues are methylated; ligating one end of the target polynucleotide to a Unique Molecular Identifier (UMI) polynucleotide and the other end of the target polynucleotide to a protective polynucleotide to form a ligated polynucleotide, wherein a portion of the UMI polynucleotide comprises a first randomly generated UMI polynucleotide sequence, wherein all of the cytosine residues of the UMI polynucleotide are unmethylated, and wherein the protective polynucleotide includes an exonuclease resistant moiety; contacting the sample with one or more exonucleases adapted to digest any polynucleotides in the sample that do not include the exonuclease resistant moiety; forming a converted polynucleotide by chemically and/or enzymatically converting each methylated cytosine in the ligated polynucleotide to dihydrouracil; amplifying the converted polynucleotide to generate a plurality of amplicon polynucleotides; sequencing the plurality of amplicon polynucleotides to generate a plurality of amplicon sequence reads. In some embodiments, each of the amplicon sequence reads: corresponds to the polynucleotide sequence of one of the plurality of amplicon polynucleotides; includes the randomly generated UMI polynucleotide sequence; and includes a thymine at each nucleotide position corresponding to the nucleotide position of a methylated cytosine in the target polynucleotide and a cytosine at each nucleotide position corresponding to an unmethylated cytosine in the target polynucleotide unless a conversion error during the conversion step, an amplification error during the amplification step or a sequencing error during the sequencing step causes the amplicon sequence read to include a nucleotide other than thymine at a nucleotide position corresponding to the nucleotide position of a methylated cytosine in the target polynucleotide or to include a nucleotide other than cytosine at a nucleotide position corresponding to the nucleotide position of an unmethylated cytosine in the target polynucleotide. The methods may further include aligning the plurality of amplicon sequencing reads with a target polynucleotide reference sequence In some embodiments, if the sequencing step generated at least five amplicon sequence reads, then the methods may further include generating a consensus polynucleotide sequence corresponding to the polynucleotide sequence of the target polynucleotide, wherein generating the consensus polynucleotide sequence comprises: identifying each 5'-C-G-3' nucleotide pair in the target polynucleotide reference sequence; comparing each amplicon sequencing read to the target polynucleotide reference sequence to determine the identity of each nucleotide in each amplicon sequencing read aligned with each cytosine in each 5'-C-G-3' nucleotide pair in the target polynucleotide reference sequence. In some embodiments, if 50% or more of the amplicon sequencing reads include a thymine at a position aligned with a cytosine in a 5'-C-G-3' nucleotide pair in the target polynucleotide reference sequence, then the methods may further include assigning a methylated cytosine to the corresponding position in the consensus polynucleotide sequence. In some embodiments, if less than 50% of the amplicon sequencing reads include a thymine at a position aligned with a cytosine in a 5'-C-G-3' nucleotide pair in the target polynucleotide reference sequence, then assigning an unmethylated cytosine to the corresponding position in the consensus polynucleotide sequence. In some embodiments, if at least 50% of the 5'-C-G-3' nucleotide pairs in the consensus polynucleotide sequence have been assigned a methylated cytosine, then the methods may further include designating the target polynucleotide as methylated, and if less than 50% of the 5'-C-G-3' nucleotide pairs in the consensus polynucleotide sequence have been assigned a methylated cytosine, then the methods may further include designating the target polynucleotide as unmethylated.

The presence of one or more methylated target polynucleotides in the biological sample indicates the detection of methylated cytosine residues. In some embodiments, the presence of one or more methylated target polynucleotides in the biological sample indicates the detection of methylated DNA.

In some embodiments, the fraction of methylated target polynucleotides is calculated by dividing the number of methylated target polynucleotides by the total number of target polynucleotides detected in the biological sample. This fraction may be used to quantify low levels of methylated DNA in the biological sample.

Similar to the methods involving chemical or enzymatic conversion of unmethylated cytosines to uracil, the methods involving conversion of methylated cytosines to dihydrouracil may comprise the use of similar exonuclease resistant modifications and exonucleases as described above. The methods also may include similar first and second patch polynucleotides for effecting ligations, as described above.

However, the methods involving conversion of methylated cytosines to dihydrouracil may comprise the use of TET enzymes and/or borane to convert the ligated polynucleotide to the converted polynucleotide.

Similar to the methods involving chemical or enzymatic conversion of unmethylated cytosines to uracil, the methods involving conversion of methylated cytosines to dihydrouracil may comprise amplification methods as described above, including, but not limited to, amplification using the polymerase chain reaction (PCR). Moreover, the target polynucleotide similarly may be from a region of a genome known to be methylated in a specific cell type, such as cancer cells selected from the group consisting of breast cancer, ovarian cancer, lung cancer, pancreatic cancer, colorectal cancer, prostate cancer, uterine cancer, bladder cancer, and liver cancer.

For the methods involving conversion of methylated cytosines to dihydrouracil, generating the consensus polynucleotide sequence may comprise assigning a methylated cytosine to the corresponding position in the consensus polynucleotide sequence if 90% or more of the amplicon sequencing reads include a thymine at a position aligned with a cytosine in a 5'-C-G-3' nucleotide pair in the target polynucleotide reference sequence, and assigning an unmethylated cytosine to the corresponding position in the consensus polynucleotide sequence if less than 90% of the amplicon sequencing reads include a thymine at a position aligned with a cytosine in a 5'-C-G-3' nucleotide pair in the target polynucleotide reference sequence. Moreover, the target polynucleotide may be designated as methylated if at least 75% of the 5'-C-G-3' nucleotide pairs in the consensus polynucleotide sequence have been assigned a methylated cytosine, and the target polynucleotide may be designated as unmethylated if less than 75% of the 5'-C-G-3' nucleotide pairs in the consensus polynucleotide sequence have been assigned a methylated cytosine.

It should be appreciated that the consensus polynucleotide may comprise a plurality of 5'-C-G-3' nucleotide pairs.

In some embodiments, methylation of cytosine residues in a plurality of target polynucleotide sequences may be detected in the same sample. For example, the plurality of target polynucleotides may comprise more than two target polynucleotides and less than 10,000 target polynucleotides, depending on the restriction enzymes used to digest the DNA in the sample.

Moreover, a plurality of copies of each target polynucleotide may be present in a sample, where each of the plurality of copies may be ligated to different UMI polynucleotides. As such, a plurality of ligated polynucleotides may be formed corresponding to each target polynucleotide, where each of these ligated polynucleotide include a different UMI polynucleotide.

Further provided herein are methods of diagnosing a patient with cancer. The method may include detecting methylation of cytosine residues in a target polynucleotide in a sample from the patient, wherein the methylation of cytosine residues is detected according to the method as detailed herein. In some embodiments, the patient is diagnosed with cancer when methylation of cytosine residues in a target polynucleotide in the sample from the patient is detected. In some embodiments, the proportion and/or position and/or pattern of methylation in the target polynucleotide indicates the patient has a particular type or stage of cancer. The stage of cancer may be, for example, stage I, II, III, or IV. In some embodiments, the cancer is selected from breast cancer, ovarian cancer, lung cancer, pancreatic cancer, colorectal cancer, prostate cancer, uterine cancer, bladder cancer, and liver cancer. In some embodiments, the method further includes treating the patient diagnosed with cancer by administering a therapy to the patient. Therapies may include, for example, chemotherapy, radiation, immunotherapy, surgical resection, or a combination thereof.

Additional aspects of the present disclosure are described in connection with the examples set forth below.

### 4. Examples

### Example 1

### Methyl Patch PCR assay

We analyzed DNA methylation data from The Cancer Genome Atlas (Saghafinia, S., et al. Cell Rep. 2018, 25, 1066-1080) and additional publicly available data from ovarian cancer (Bartlett, T.E., et al. PLoS One 2015, 10, e0143178) and normal ovarian tissues (Klinkebiel, D., et al. Mol. Cancer Res. 2016, 14, 787-794; Patch, A.M., et al. Nature 2015, 521, 489-494), as well as publicly available datasets of DNA methylation in blood and tissue from patients with other non-malignant diseases including rheumatoid arthritis (Liu, Y., et al. Nat. Biotechnol. 2013, 31, 142-147), HIV infection (Zhang, X., et al. Epigenetics 2016, 11, 750-760), nonalcoholic fatty liver disease (Ahrens, M., et al. Cell Metab. 2013, 18, 296-302), obesity (Kirchner, H., et al. Mol. Metab. 2016, 5, 171-183), type-2 diabetes (Kirchner, H., et al. Mol. Metab. 2016, 5, 171-183), and inflammatory bowel disease (Ventham, N.T., et al. Nat. Commun. 2016, 7, 13507). We used statistical analysis to identify CG dinucleotide positions (5'-C-G-3' dinucleotides or CGs) in the human genome reference sequence that were methylated in breast cancer, ovarian cancer, uterine cancer, pancreatic cancer, colon cancer, rectal cancer, liver cancer, lung cancer, prostate cancer, and/or bladder cancer, and unmethylated in normal tissue and blood from patients without cancer. We also selected CG dinucleotide positions that were methylated in blood cells but not methylated in tumors and normal tissues. We then computationally analyzed 400bp of polynucleotide reference genome sequence surrounding these CG positions to determine if restriction digest of DNA with different pairs of methyl-insensitive enzymes would produce DNA fragments greater than 37bp less than 175bp in length that contain the target CG and at least 2 other CGs. We selected 219 CGs that met this criteria, including 8 CGs that were methylated across cancers, 70 CGs that were methylated primarily in breast cancer, 9 CGs that were primarily methylated in lung cancer, 38 CGs that were methylated primarily in ovarian cancer, 54 CGs that were methylated primarily in uterine cancer, 33 CGs that were methylated primarily in pancreatic cancer, 58 CGs that were methylated primarily in colon cancer, 57 CGs that were methylated primarily in rectal cancer, 35 CGs that were methylated primarily in liver cancer, 24 CGs that were methylated primarily in lung adenocarcinoma, 7 CGs that were methylated primarily in lung squamous carcinoma, 28 CGs that were methylated primarily in prostate cancer, 47 CGs that were methylated primarily in bladder cancer, and 9 CGs that were methylated in blood cells but not methylated in tumors and normal tissues (**FIG. 4****).**

The median number of CGs in the target genomic regions is 10 CGs, which is consistent with previous studies that have demonstrated that hypermethylation in cancer occurs at CG islands, which are regions of the genome enriched for CG dinucleotides (Varley, K.E., et al. Genome Res. 2013, 23, 555-567; Toyota, M. and J.P. Issa. Electrophoresis 2000, 21, 329-333). DNMT3A is the enzyme that catalyzes de novo DNA in mammalian cells, and its activity is processive, which results in the methylation of multiple CGs in close proximity in the genome. This aspect of DNA methylation provides an advantage for detecting rare methylated molecules because if there are multiple CGs within the same genomic fragment, we expect more than one of the CGs to be methylated.

Next, we designed patch oligos to capture 183 regions of the genome that contain the 219 target CGs we selected. As described in our previous studies (Varley, K.E. and R.D. Mitra. Genome Res. 2010, 20, 1279-1287; Varley, K.E. and R.D. Mitra. Genome Res. 2008, 18, 1844-1850; Varley, K.E. and R.D. Mitra. Cold Spring Harb. Protoc. 2009, 2009, pdb prot5252), we performed an *in silico* digest of polynucleotide reference genome sequence to identify a pair of methyl-insensitive restriction enzymes that create target polynucleotide restriction fragments that contain the target CG positions. For this panel we chose Ddel and HpyCH4V. We designed patch oligos to anneal specifically to the ends of the target polynucleotide fragments with a Tm of 60-70°C, which provides highly specific annealing, similar to PCR primers. The patch oligos are also composed of sequence that allows them to anneal to universal adapters, leaving only a break in the backbone between the target DNA fragment and the universal adapter **(****FIG. 5****).** The left universal adapter is designed to contain a Unique Molecular Identifier (UMI) in the Index 2 position, which is synthesized to contain a random series of 12 bases composed of 5-methyl-C, G, A or T at each position with equal probability, providing 16,777,216 possible barcodes to uniquely identify individual template molecules. The right universal adapter is synthesized to contain a 3' 3 carbon spacer and 3 phosphorothioate bonds, which protects the molecule from degradation during subsequent exonuclease treatment **(****FIG. 5****).** Thermostable ligase is used to covalently close the break in the DNA backbone between the universal adapters and the ends of the 183 target polynucleotide DNA fragments in a single reaction **(****FIG. 5****).** This ligation reaction is cycled between melting and annealing 25 times to provide multiple chances for target DNA molecules to anneal properly to a patch oligo and be ligated to the universal adapters. Following ligation of the universal adapters, the reaction is treated with two 3'-5' exonucleases (Exonuclease I and Exonuclease III), which degrade untargeted DNA and patch oligos **(****FIG. 5****).** Target polynucleotide DNA molecules are protected from digestion by the exonuclease modification at the 3' end of the right universal adapter.

After the target polynucleotide DNA fragments are ligated to the universal adapters, and untargeted DNA is degraded by exonuclease, chemical or enzymatic conversion of cytosine is performed to enable detection of DNA methylation by sequencing **(****FIG. 5****).** We have tested several conversion methods at this step including sodium bisulfite conversion (Frommer, M., et al. Proc. Natl. Acad. Sci. U.S.A. 1992, 89, 1827-1831), enzymatic conversion with TET and APOBEC enzymes (Louise Williams, P.D., et al. Enzymatic Methyl-seq: The Next Generation of Methylome Analysis Available from: https://www.neb.com/tools-and-resources/feature-articles/enzymatic-methyl-seq-the-next-generation-of-methylome-analysis), and enzymatic conversion with TET enzymes followed by deamination with pyridine borane (Liu, Y., et al. Nat. Biotechnol. 2019, 37, 424-429). A comparison of template degradation and conversion efficiency is described below.

Following chemical or enzymatic conversion of cytosine in the ligated polynucleotides, PCR amplification of the converted polynucleotide is performed using a left universal primer and a right universal primer that includes a sample-specific DNA barcode sequence **(****FIG. 5****).** PCR products (amplicon polynucleotides) from multiple samples are purified and pooled for sequencing on a next-generation sequencing instrument. In the results described below, the PCR products are sequenced on illumina MiSeq and illumina NovaSeq instruments.

### Example 2

### Methyl Patch PCR sequencing analysis

Sequencing data from each sample was separated into different files (demultiplexed) using the sample-specific barcode sequenced added during the final PCR reaction according to standard procedures known to those skilled in the art.

The amplicon sequence reads from each sample were then aligned to a custom reference genome containing the sequence of the target genomic regions, or the whole genome, using a bioinformatics algorithm that accounts for the conversion of cytosine in the amplicon sequence reads. In this study we used the bioinformatics software Bismark (Krueger, F. and S.R. Andrews, Bioinformatics 2011, 27, 1571-1572) to align the reads to the target genomic regions.

### Example 3

### Capture efficiency

To evaluate the performance of the capture method, we calculate how many of our target polynucleotide sequences are covered by the amplicon sequence reads. In the experiments described below, greater than 90% of the target regions were successfully captured, amplified and sequenced. We also calculate the percentage of amplicon sequence reads that align to the target polynucleotide sequences. In the experiments described below, the percentage of amplicon sequence reads that align to the target genomics regions is greater than 85%, with an average of 90%. These results indicate that the Methyl Patch PCR assay is highly sensitive and specific for capturing the target regions of the genome.

### Example 4

### Comparison of cytosine conversion methods

DNA polymerases do not copy the methylation state of the template DNA molecules. In order to distinguish 5-methyl-cytosine from unmethylated cytosine in amplicon sequence reads, chemical or enzymatic conversion of the cytosine base must be performed before PCR. The current gold standard approach is bisulfite sequencing where the DNA is treated with sodium bisulfite to convert unmethylated cytosine to uracil, leaving methylated cytosine intact (Frommer, M., et al. Proc. Natl. Acad. Sci. U.S.A. 1992, 89, 1827-1831). During subsequent PCR amplification uracil is copied as thymine and methylated cytosine is copied as cytosine. It is known that sodium bisulfite conversion is necessarily a harsh treatment, which causes extensive DNA damage in the form of nicks, fragmentation, and abasic sites (Ehrich, M., et al., Nucleic Acids Res. 2007, 35, e29; Grunau, C., et al. Nucleic Acids Res. 2001, 29, E65-E65). An alternative approach was recently developed that uses TET2 to enzymatically oxidize 5-methyl cytosine through a cascade reaction into 5-carboxycytosine (5caC) (Louise Williams, P.D., et al. Enzymatic Methyl-seq: The Next Generation of Methylome Analysis Available from: https://www.neb.com/tools-and-resources/feature-articles/enzymatic-methyl-seq-the-next-generation-of-methylome-analysis). The converted DNA is then treated with APOBEC, which enzymatically deaminates cytosine to uracil, but does not affect 5caC (Louise Williams, P.D., et al. Enzymatic Methyl-seq: The Next Generation of Methylome Analysis Available from: https://www.neb.com/tools-and-resources/feature-articles/enzymatic-methyl-seq-the-next-generation-of-methylome-analysis). These enzymatic treatments result in the same amplicon sequence changes as sodium bisulfite conversion: unmethylated cytosine is read as thymine, and methylated cytosine is read as cytosine. This enzymatic method is reportedly less damaging to DNA (Louise Williams, P.D., et al. Enzymatic Methyl-seq: The Next Generation of Methylome Analysis Available from: https://www.neb.com/tools-and-resources/feature-articles/enzymatic-methyl-seq-the-next-generation-of-methylome-analysis).

The detection of low levels (<1%) of methylated DNA requires deep sequencing of target polynucleotide molecules. We performed an experiment to compare the number of molecules that were sequenced when Methyl Patch PCR was performed on 100ng of genomic DNA with no cytosine conversion, sodium bisulfite conversion (Zymo Research EZ DNA Methylation-Lightning Kit), and enzymatic cytosine conversion (New England Biolabs NEBNext Enzymatic Methyl-seq Conversion Module). We analyzed the sequencing results from this experiment, and counted the number of unique molecular identifiers with at least 5 sequencing reads for each of the target genomic regions. We found that sodium bisulfite conversion resulted in significantly fewer unique molecules sequenced compared to the enzymatic cytosine conversion **(****FIG. 6****).** To maximize the number of molecules sequenced, and thereby increase our ability to detect low levels of methylated DNA, we choose to use enzymatic cytosine conversion in subsequent experiments.

### Example 5

### Cis-CG analysis to detect low levels of methylated DNA

To evaluate and optimize the detection of low levels of methylated ctDNA we performed the Methyl Patch PCR assay on 100ng of DNA composed of methylated DNA diluted into DNA isolated from healthy donor plasma at the following percentages: 1%, 0.75%, 0.5%, 0.25%, 0.1%, and 0%. We used this set of samples to determine the optimal bioinformatics approach for quantifying low levels of methylated DNA **(****FIG. 7****).**

We first analyzed the sequencing results to determine if counting amplicon sequence reads that appeared methylated at the single target CG identified from our analysis of TCGA data was sufficient to accurately detect low levels of methylation. We found that the number of reads that appeared methylated at the target CG position was significantly higher than expected, and there was no significant difference in the methylation levels between the samples with 1% and 0.1% methylated DNA **(****FIG. 8****).** This result is likely due to PCR and sequencing errors introducing Cs into the sequencing reads inappropriately, creating false-positives that appear methylated. This result demonstrated that counting reads that appeared methylated at the single target CG was inadequate for quantifying low levels of methylated DNA.

To reduce the false-positive methylation calls introduced by PCR and sequencing errors in the amplicon sequence reads, we used the unique molecular identifier (UMI) to perform error correction. For each UMI that had at least 5 sequencing reads, we calculated the percent of reads that contained a cytosine at the single target CG position, as depicted in **FIG. 7****.** A consensus call threshold is used to determine whether a high enough percentage of amplicon sequence reads contain a cytosine at the target position to be confident that the molecule was methylated, or whether only a fraction of reads contain a cytosine at the target position that were introduced through PCR and sequencing errors.

To determine the optimal consensus call threshold to accurately identify methylated molecules, we tested a range of consensus call thresholds (10-100%). For each sample in the titration experiment, we calculated the percent of molecules that appeared methylated at the target CG using different consensus call thresholds (10-100%). We compared the percentage of methylated molecules observed using each threshold to the expected values in the titration experiment **(****FIG. 9****).**

We found that increasing the thresholds from 10% to 90% consensus reduced the number of molecules that appeared methylated to more closely match the expected values based on the titration **(****FIG. 9****),** indicating that PCR and sequencing errors were introducing false-positive results in our previous analysis. Requiring 100% of the reads to contain a cytosine at the target CG position made the observed methylation level most closely matched the expected value for the sample containing 1% methylation, however this threshold also resulted in flattening the titration curve and diminishing the difference in methylation observed between the samples containing 1% and 0% methylation **(****FIG. 9****).** This result is likely due to two confounding factors. The first is low levels (approximately 1%) of incomplete conversion of the unmethylated DNA, creating false-positives that appear methylated across all samples. The second is a reduced observation of expected methylation because PCR and sequencing errors that revert cytosine to a thymine in the amplicon sequencing reads result in the molecule failing to meet the 100% threshold, producing false-negatives. The net result is that we primarily observe false-positive methylation due to incomplete conversion, and fail to detect low levels of methylated DNA in the titration because of over-correction during the consensus call. For this reason, we chose a consensus call threshold that requires >=90% of the reads to appear methylated at the target CG position. This approach allows for PCR and sequencing errors that reverts methylated cytosine to a thymine at rates <10%.

Using this >=90% consensus call threshold, we found that the number of unique molecules with methylated consensus calls at the target CG was higher than expected across the titration (**FIG. 8** and **FIG. 9**). This result is likely due to low levels (<1%) of incomplete conversion of the unmethylated DNA, creating false-positives that appear methylated. This result demonstrated that counting molecules that appeared methylated at the target CG after UMI-based error correction was inadequate for quantifying low levels of methylated DNA because this approach over-estimates the amount of methylation in the sample.

Cytosine conversion methods are not 100% efficient, and incomplete conversion of unmethylated cytosine will create false-positive methylation calls at individual CG positions. One advantage of the Methyl Patch PCR assay is that we can capture target regions that contain multiple CG positions, and based upon the biological processivity of DNA methyltransferases and the genomic structure of CpG islands, we expect CGs that are in close proximity in the genome to be coordinately methylated. To reduce the false-positive methylation calls introduced by incomplete conversion of individual cytosine positions, we developed a method to analyze all the cytosines in the target genomic region, rather than analyzing a single target CG in the region.

The first step of the analysis uses the UMI-based error correction approach described above, wherein each UMI that has at least 5 sequencing reads is analyzed to calculate a consensus call for each CG in the molecule **(****FIG. 7****).** As described above, we use a consensus call threshold to determine that each CG in a target polynucleotide reference sequence is methylated if >90% of the reads contained a cytosine at that CG position. This results in a consensus polynucleotide sequence for each UMI, that is, the template molecule.

In the second step of the analysis, we determine whether multiple CGs across the molecule are methylated according to the consensus sequence **(****FIG. 7****).** This is referred to as cis-CG analysis. A cis-CG threshold is used to determine whether the consensus polynucleotide sequence for each UMI contains enough cytosines at CGs positions to be confident that the molecule was methylated, or whether only a fraction of CGs in the consensus polynucleotide sequence are cytosines that remained due to incomplete conversion. To determine the optimal cis-CG threshold for accurately identifying methylated molecules, we tested a range of cis-CG thresholds (5-100%). For each sample in the titration experiment, we calculated the percent of UMIs that appeared methylated based on different cis-CG thresholds (5-100%). We compared the percentage of methylated UMIs observed using each threshold to the expected values in the titration experiment **(****FIG. 10****).** We found that low thresholds (5-50%) made it appear that there were significantly more methylated molecules than expected in the titration samples, indicating that incomplete conversion errors introduced false-positives **(****FIG. 10****).** In contrast, the high thresholds (85-100%) made it appear that there were significantly fewer methylated molecules than expected in the titration samples, indicating that rare inappropriate conversion of methylated cytosines may create false-negative results **(****FIG. 10****).** We found that a cis-CG threshold that requires >=75% of the CGs in the consensus polynucleotide sequence for each UMI to appear methylated provided the most accurate estimate of the percent of methylated molecules for the samples in the titration **(****FIG. 10****).**

Using the >=90% consensus call threshold, and the >=75% cis-CG threshold, we found that the percent of methylated molecules observed in the titration experiment was closer to the expected frequency across the titration range of methylated DNA (1% through 0.1%) **(****FIG. 8****).** This result demonstrated that using UMI-based error correction followed by counting molecules that appeared methylated across multiple CGs was the most effective strategy for quantifying low levels of methylated DNA.

### Example 6

### Evaluating methylation in human patient samples

Next, we sought to determine if the Methyl Patch PCR assay, UMI error correction, and Cis-CG analysis could be used to detect cancer-specific DNA methylation in human cancer samples. We performed the Methyl Patch PCR assay on 100ng of DNA isolated from 5 breast cancer tumors, 7 ovarian cancer tumors, 1 endometrial (uterine) cancer tumor, 2 colorectal cancer tumors, 1 bladder tumor, 1 lung tumor, and 1 prostate tumor. We calculated the fraction of molecules that appeared methylated across each target genomic region (polynucleotide) in each sample **(****FIG. 11****).**

We found that each tumor type exhibited a distinct pattern of DNA methylation across the target regions **(****FIG. 11****).** This panel contained target regions that were selected because they contained a CG position that exhibited higher methylation in one particular cancer type in the TCGA data. Our data demonstrate that each tumor type is enriched for methylation at target regions that contained CGs that were methylated specifically in each corresponding cancer type in TCGA data **(****FIG. 11****).** This is an important confirmation of our approach because the TCGA data only reported methylation at a single CG position in the target polynucleotide, whereas our cis-CG analysis requires that multiple CGs in each target polynucleotide be methylated in order for the molecule to be counted as methylated. This result indicates that our approach can be used to detect cancer type-specific DNA methylation in human samples.

To determine if the detection of DNA methylation at cancer-type specific target genomic regions in each sample could be used to predict tumor type, we calculated the mean (average) fraction of methylated molecules observed in each sample across breast target regions, gynecologic target regions (including both ovarian and uterine target regions), colorectal target regions, bladder target regions, lung target regions, liver target regions, pancreatic target regions, and prostate target regions. In each sample, the highest mean fraction methylated corresponded to the cancer-type for that sample **(****FIG. 11****).** In all 5 of the breast cancer tumor samples, the set of target regions with the highest mean fraction methylated was the breast target regions **(****FIG. 11****).** In the 7 gynecologic cancer samples that included ovarian and endometrial cancers, the set of target regions with the highest mean fraction methylated was the gynecologic target regions **(****FIG. 11****).** In the 2 colorectal cancer samples, the set of target regions with the highest mean fraction methylated was the colorectal target regions **(****FIG. 11****).** The bladder target regions had the highest mean fraction methylated in the bladder cancer; the lung target regions had the highest mean fraction methylated in the lung cancer; and the prostate target regions had the highest mean fraction methylated in the prostate cancer **(****FIG. 11****).** This limited dataset indicates that we can predict tumor type with 100% accuracy **(****FIG. 11****).** This result confirms that our approach can be used to detect DNA methylation that is primarily found in specific types of human cancer.

This experiment illustrates how the multiplexed nature of the Methyl Patch PCR assay and Cis-CG analysis allows multiple genomic loci to be queried for low levels of DNA methylation simultaneously in the same sample, and how this information can be used to determine what type of tumor the DNA came from.

### Example 7

### Detecting low levels of methylated tumor DNA in blood plasma samples

To determine if our approach could be used to detect methylated ctDNA, we performed the Methyl Patch PCR assay, UMI error correction, and Cis-CG analysis on three sets of samples. The first set of samples included 3 independent replicate titrations of methylated DNA diluted into DNA isolated from healthy donor blood plasma at the following percentages: 1%, 0.75%, 0.5%, 0.25%, 0.1%, 0%. The second set of samples included DNA isolated from blood plasma from 16 healthy donors. The third set of samples included DNA isolated from blood plasma collected from 13 breast cancer patients in the time interval between diagnosis and surgical resection of their tumors. We calculated the fraction of methylated UMIs (molecules) observed for each target region in each sample.

To provide the most accurate quantification of the fraction of methylated molecules for each target regions, we used linear regression to calculate the coefficient (slope) that provided the best fit line between the observed fraction methylated and expected methylation across the three replicate titrations. We then multiplied the coefficients for each target region by the fraction methylated values observed for that region in each healthy donor, breast cancer patient, and titration sample. This approach generates an adjusted fraction methylated value and is analogous to fitting values to a standard curve.

To identify positive ctDNA measurements and distinguish them from normal methylation observed in blood plasma and noise in the assay, we performed background subtraction. For each target region, we calculated the adjusted fraction methylated value that is the 95th percentile across the 16 healthy donor samples, and subtracted this value from each adjusted fraction methylated value for each target region in each sample to generate a normalized fraction methylated value. The normalized fraction methylated values that remained above zero were considered positives.

In the titrations of methylated DNA into healthy donor plasma DNA, the mean of the normalized fraction methylated values consistently decreased across the range methylated DNA inputs (1%-0%) **(****FIG. 12****),** indicating that we can quantify low levels (<1%) of methylated DNA in cell-free DNA isolated from blood plasma. We observed fewer positive values across the healthy donor plasma samples compared to the breast cancer patient plasma samples **(****FIG. 12****).** The mean of the normalized fraction methylated values in each of the healthy donor samples was lower than 0.15, and lower than all of the breast cancer patient plasma samples **(****FIG. 12****).** The breast cancer patient plasma samples all produced mean normalized fraction methylated values greater than 0.15 **(****FIG. 12****).** The presence of higher quantities of methylated DNA in all of the breast cancer samples compared to all of the healthy donor samples confirms that our approach can detect low levels of methylated ctDNA in blood plasma samples from patients with cancer.

We compared the mean normalized fraction methylated values between the breast cancer patients and the titrations. We observe that 7/13 of the breast cancer patients have mean normalized fraction methylated values within the range of values observed in the titration. This indicates that these patients had 0.1-1% methylated DNA in their blood plasma. The remaining 6/13 breast cancer patients had higher mean normalized fraction methylated values than the titration range, indicating they had more than 1% methylated DNA in their blood plasma. This result indicates that detection and quantification of low levels of methylated DNA is necessary to detect ctDNA in 54% (7/13) of breast cancer patients in this study.

Notably, four of the breast cancer patients were diagnosed with Stage I disease. We were able to detect methylated ctDNA in all four of these patients with early stage disease. The mean normalized fraction methylated values observed in 2 of these patients lie within the range of the titrations, indicating that detection and quantification of low levels of methylated DNA (<1%) is necessary to detect ctDNA in 50% (2/4) of early stage breast cancer patients in this study.

## Claims

1. A method of detecting methylation of cytosine residues in a target polynucleotide in a sample comprising DNA, the method comprising:
digesting the DNA in the sample with a methyl-insensitive restriction enzyme to create a target polynucleotide comprising a plurality of cytosine residues, wherein one or more of the cytosine residues are methylated;
ligating one end of the target polynucleotide to a Unique Molecular Identifier (UMI) polynucleotide and the other end of the target polynucleotide to a protective polynucleotide to form a ligated polynucleotide, wherein a portion of the UMI polynucleotide comprises a first randomly generated UMI polynucleotide sequence, wherein all of the cytosine residues of the UMI polynucleotide are methylated, and wherein the protective polynucleotide includes an exonuclease resistant moiety;
contacting the sample with one or more exonucleases adapted to digest any polynucleotides in the sample that do not include the exonuclease resistant moiety;
forming a converted polynucleotide by chemically and/or enzymatically converting each unmethylated cytosine in the ligated polynucleotide to uracil;
amplifying the converted polynucleotide to generate a plurality of amplicon polynucleotides;
sequencing the plurality of amplicon polynucleotides to generate a plurality of amplicon sequence reads, wherein each of the amplicon sequence reads:
corresponds to the polynucleotide sequence of one of the plurality of amplicon polynucleotides;
includes the randomly generated UMI polynucleotide sequence; and
includes a thymine at each nucleotide position corresponding to the nucleotide position of an unmethylated cytosine in the target polynucleotide and a cytosine at each nucleotide position corresponding to a methylated cytosine in the target polynucleotide unless a conversion error during the conversion step, an amplification error during the amplification step, or a sequencing error during the sequencing step causes the amplicon sequence read to include a nucleotide other than thymine at a nucleotide position corresponding to the nucleotide position of an unmethylated cytosine in the target polynucleotide or to include a nucleotide other than cytosine at a nucleotide position corresponding to the nucleotide position of a methylated cytosine in the target polynucleotide; and
aligning the plurality of amplicon sequencing reads with a target polynucleotide reference sequence;
if the sequencing step generated at least five amplicon sequence reads, then generating a consensus polynucleotide sequence corresponding to the polynucleotide sequence of the target polynucleotide, wherein generating the consensus polynucleotide sequence comprises:
identifying each 5'-C-G-3' nucleotide pair in the target polynucleotide reference sequence;
comparing each amplicon sequencing read to the target polynucleotide reference sequence to determine the identity of each nucleotide in each amplicon sequencing read aligned with each cytosine in each 5'-C-G-3' nucleotide pair in the target polynucleotide reference sequence;
if 50% or more of the amplicon sequencing reads include a cytosine at a position aligned with a cytosine in a 5'-C-G-3' nucleotide pair in the target polynucleotide reference sequence, then assigning a methylated cytosine to the corresponding position in the consensus polynucleotide sequence; and
if less than 50% of the amplicon sequencing reads include a cytosine at a position aligned with a cytosine in a 5'-C-G-3' nucleotide pair in the target polynucleotide reference sequence, then assigning an unmethylated cytosine to the corresponding position in the consensus polynucleotide sequence; and
wherein if at least 50% of the 5'-C-G-3' nucleotide pairs in the consensus polynucleotide sequence have been assigned a methylated cytosine, then designating the target polynucleotide as methylated, and wherein if less than 50% of the 5'-C-G-3' nucleotide pairs in the consensus polynucleotide sequence have been assigned a methylated cytosine, then designating the target polynucleotide as unmethylated.

2. A method of detecting methylation of cytosine residues in a target polynucleotide in a sample comprising DNA, the method comprising:
digesting the DNA in the sample with a methyl-insensitive restriction enzyme to create a target polynucleotide comprising a plurality of cytosine residues, wherein one or more of the cytosine residues are methylated;
ligating one end of the target polynucleotide to a Unique Molecular Identifier (UMI) polynucleotide and the other end of the target polynucleotide to a protective polynucleotide to form a ligated polynucleotide, wherein a portion of the UMI polynucleotide comprises a first randomly generated UMI polynucleotide sequence, wherein all of the cytosine residues of the UMI polynucleotide are unmethylated, and wherein the protective polynucleotide includes an exonuclease resistant moiety; contacting the sample with one or more exonucleases adapted to digest any polynucleotides in the sample that do not include the exonuclease resistant moiety;
forming a converted polynucleotide by chemically and/or enzymatically converting each methylated cytosine in the first ligated polynucleotide to dihydrouracil;
amplifying the converted polynucleotide to generate a plurality of amplicon polynucleotides;
sequencing the plurality of amplicon polynucleotides to generate a plurality of amplicon sequence reads, wherein each of the amplicon sequence reads:
corresponds to the polynucleotide sequence of one of the plurality of amplicon polynucleotides;
includes the randomly generated UMI polynucleotide sequence; and
includes a thymine at each nucleotide position corresponding to the nucleotide position of a methylated cytosine in the target polynucleotide and a cytosine at each nucleotide position corresponding to an unmethylated cytosine in the target polynucleotide unless a conversion error during the conversion step, an amplification error during the amplification step or a sequencing error during the sequencing step causes the amplicon sequence read to include a nucleotide other than thymine at a nucleotide position corresponding to the nucleotide position of a methylated cytosine in the target polynucleotide or to include a nucleotide other than cytosine at a nucleotide position corresponding to the nucleotide position of an unmethylated cytosine in the target polynucleotide; and
aligning the plurality of amplicon sequencing reads with a target polynucleotide reference sequence;
if the sequencing step generated at least five amplicon sequence reads, then generating a consensus polynucleotide sequence corresponding to the polynucleotide sequence of the target polynucleotide, wherein generating the consensus polynucleotide sequence comprises:
identifying each 5'-C-G-3' nucleotide pair in the target polynucleotide reference sequence;
comparing each amplicon sequencing read to the target polynucleotide reference sequence to determine the identity of each nucleotide in each amplicon sequencing read aligned with each cytosine in each 5'-C-G-3' nucleotide pair in the target polynucleotide reference sequence;
if 50% or more of the amplicon sequencing reads include a thymine at a position aligned with a cytosine in a 5'-C-G-3' nucleotide pair in the target polynucleotide reference sequence, then assigning a methylated cytosine to the corresponding position in the consensus polynucleotide sequence; and
if less than 50% of the amplicon sequencing reads include a thymine at a position aligned with a cytosine in a 5'-C-G-3' nucleotide pair in the target polynucleotide reference sequence, then assigning an unmethylated cytosine to the corresponding position in the consensus polynucleotide sequence; and
wherein if at least 50% of the 5'-C-G-3' nucleotide pairs in the consensus polynucleotide sequence have been assigned a methylated cytosine, then designating the target polynucleotide as methylated, and wherein if less than 50% of the 5'-C-G-3' nucleotide pairs in the consensus polynucleotide sequence have been assigned a methylated cytosine, then designating the target polynucleotide as unmethylated.

3. The method of claims 1 or 2, the method further comprising calculating the fraction of methylated target polynucleotides in the sample by dividing the number of methylated target polynucleotides by the total number of target polynucleotides detected in the sample.

4. The method of claims 1 or 2, wherein the exonuclease resistant modification comprises a phosphorothioate bond and/or a 3-carbon spacer.

5. The method of either claim 1 or 2 or 3 or 4, wherein ligating the target polynucleotide to the UMI polynucleotide comprises annealing a first patch polynucleotide to both the target polynucleotide and the UMI polynucleotide, and wherein ligating the target polynucleotide to the protective polynucleotide comprises annealing a second patch polynucleotide to both the target polynucleotide and the protective polynucleotide.

6. The method of any of claims 1-5, wherein TET2 and APOBEC are used to enzymatically convert the ligated polynucleotide to the converted polynucleotide.

7. The method of any of claims 1-6, wherein the converted polynucleotide is amplified using the polymerase chain reaction (PCR).

8. The method of any of claims 1-7, wherein the target polynucleotide is from a region of a genome known to be methylated in a specific cell type, optionally wherein the specific cell type is a specific type of cancer cell, further optionally wherein the specific type of cancer cell is a cancer cell selected from the group consisting of breast cancer, ovarian cancer, lung cancer, pancreatic cancer, colorectal cancer, prostate cancer, uterine cancer, bladder cancer, and liver cancer.

9. The method of any of claims 1-8, wherein generating the consensus polynucleotide sequence comprises assigning a methylated cytosine to the corresponding position in the consensus polynucleotide sequence if 90% or more of the amplicon sequencing reads include a cytosine at a position aligned with a cytosine in a 5'-C-G-3' nucleotide pair in the target polynucleotide reference sequence, and assigning an unmethylated cytosine to the corresponding position in the consensus polynucleotide sequence if less than 90% of the amplicon sequencing reads include a cytosine at a position aligned with a cytosine in a 5'-C-G-3' nucleotide pair in the target polynucleotide reference sequence.

10. The method of any of claims 1-8, wherein the target polynucleotide is designated as methylated if at least 75% of the 5'-C-G-3' nucleotide pairs in the consensus polynucleotide sequence have been assigned a methylated cytosine, and the target polynucleotide is designated as unmethylated if less than 75% of the 5'-C-G-3' nucleotide pairs in the consensus polynucleotide sequence have been assigned a methylated cytosine.

11. The method of any of claims 1-10, wherein the consensus polynucleotide comprises a plurality of 5'-C-G-3' nucleotide pairs.

12. The method of any of claims 1-11, wherein methylation of cytosine residues in a plurality of target polynucleotide sequences are detected in the same sample, optionally, wherein the plurality of target polynucleotides comprises more than two target polynucleotides and less than 10,000 target polynucleotides.

13. A method of diagnosing a patient with cancer, the method comprising detecting methylation of cytosine residues in a target polynucleotide in a sample from the patient, wherein the methylation of cytosine residues is detected according to the method of any one of claims 1-12 and wherein the patient is diagnosed with cancer when methylation of cytosine residues in a target polynucleotide in the sample from the patient is detected.

14. The method of claim 13, wherein the cancer is selected from the group consisting of breast cancer, ovarian cancer, lung cancer, pancreatic cancer, colorectal cancer, prostate cancer, uterine cancer, bladder cancer, and liver cancer.

15. The method of claim 13 or 14, the method further indicating how to treat the patient diagnosed with cancer by chemotherapy, radiation, immunotherapy, surgical resection, or a combination thereof.

## Patentansprüche

1. Verfahren zum Detektieren der Methylierung von Cytosinresten in einem Target-Polynucleotid in einer Probe, die DNA umfasst, wobei das Verfahren Folgendes umfasst:
Digerieren der DNA in der Probe mit einem gegen Methyl unempfindlichen Restriktionsenzym, um ein Target-Polynucleotid zu erzeugen, das eine Vielzahl von Cytosinresten umfasst, wobei ein oder mehrere der Cytosinreste methyliert sind;
Ligieren eines Endes des Target-Polynucleotids an ein Eindeutiges Molekulares Identifikator- (UMI-) Polynucleotid und des anderen Endes des Target-Polynucleotids an ein schützendes Polynucleotid, um ein ligiertes Polynucleotid zu bilden, wobei ein Teil des UMI-Polynucleotids eine erste willkürlich erzeugte UMI-Polynucleotidsequenz umfasst, wobei alle der Cytosinreste des UMI-Polynucleotids methyliert werden und wobei das schützende Polynucleotid einen exonucleaseresistenten Anteil einschließt,
Kontaktieren der Probe mit einer oder mehreren Exonucleasen, die zum Digerieren irgendwelcher Polynucleotide in der Probe, die den exonucleaseresistenten Anteil nicht einschließen, geeignet sind;
Bilden eines umgewandelten Polynucleotids durch chemisches und/oder enzymatisches Umwandeln jedes unmethylierten Cytosins in dem ligierten Polynucleotid in Uracil;
Amplifizieren des umgewandelten Polynucleotids, um eine Vielzahl von Amplikonpolynucleotiden erzeugen,
Sequenzieren der Vielzahl von Amplikonpolynucleotiden, um eine Vielzahl von Amplikonsequenz-Reads zu erzeugen, wobei jedes von den Amplikonsequenz-Reads:
der Polynucleotidsequenz von einem von der Vielzahl von Amplikonpolynucleotiden entspricht,
die willkürlich erzeugte UMI-Polynucleotidsequenz einschließt, und
ein Thymin an jeder Nucleotidposition, die der Nucleotidposition eines unmethylierten Cytosins in dem Target-Polynucleotid entspricht, und ein Cytosin an jeder Nucleotidposition, die einem methylierten Cytosin in dem Target-Polynucleotid entspricht, einschließt, es sei denn ein Umwandlungsfehler während des Umwandlungsschritts, ein Amplifikationsfehler während des Amplifikationsschritts oder ein Sequenzierfehler während des Sequenzierschritts verursacht, dass das Amplikonsequenz-Read ein Nucleotid einschließt, bei dem es sich nicht um Thymin an einer Nucleotidposition handelt, die der Nucleotidposition eines unmethylierten Cytosins in dem Target-Polynucleotid entspricht oder ein Nucleotid einschließt, bei dem es sich nicht um Cytosin an einer Nucleotidposition handelt, die der Nucleotidposition eines methylierten Cytosins in der Target-Polynucleotid entspricht, und
Ausrichten der Vielzahl von Amplikonsequenz-Reads auf eine Target-Polynucleotid-Referenzsequenz;
wenn der Sequenzierschritt mindestens fünf Amplikonsequenz-Reads erzeugt hat, dann Erzeugen einer Konsens-Polynucleotidsequenz, die der Polynucleotidsequenz des Target-Polynucleotids entspricht, wobei das Erzeugen der Konsens-Polynucleotidsequenz Folgendes umfasst:
Identifizieren jedes 5'-C-G-3'-Nucleotidpaars in der Target-Polynucleotid-Referenzsequenz,
Vergleichen jedes Amplikonsequenz-Reads mit der Target-Polynucleotid-Referenzsequenz, um die Identität jedes Nucleotids in jedem Amplikonsequenzier-Read zu bestimmen, das auf jedes Cytosin in jedem 5'-C-G-3'-Nucleotidpaar in der Target-Polynucleotid-Referenzsequenz ausgerichtet ist,
wenn 50 % oder mehr der Amplikonsequenzier-Reads ein Cytosin an einer Position einschließen, die auf ein Cytosin in einem 5'-C-G-3'-Nucleotidpaar in der Target-Polynucleotid-Referenzsequenz ausgerichtet ist, dann Zuweisen eines methylierten Cytosins zu der entsprechenden Position in der Konsens-Polynucleotidsequenz; und
wenn weniger als 50 % der Amplikonsequenzier-Reads ein Cytosin an einer Position einschließen, die auf ein Cytosin in einem 5'-C-G-3'-Nucleotidpaar in der Target-Polynucleotid-Referenzsequenz ausgerichtet ist, dann Zuweisen eines unmethylierten Cytosins zu der entsprechenden Position in der Konsens-Polynucleotidsequenz,; und
wobei, wenn mindestens 50 % der 5'-C-G-3'-Nucleotidpaare in der Konsens-Polynucleotidsequenz ein methyliertes Cytosin zugewiesen worden ist, dann Designieren des Target-Polynucleotids als methyliert, und wobei, wenn weniger als 50 % der 5'-C-G-3'-Nucleotidpaare in der Konsens-Polynucleotidsequenz ein methyliertes Cytosin zugewiesen worden ist, dann Designieren des Target-Polynucleotids als unmethyliert.

2. Verfahren zum Detektieren der Methylierung von Cytosinresten in einem Target-Polynucleotid in einer Probe, die DNA umfasst, wobei das Verfahren Folgendes umfasst:
Digerieren der DNA in der Probe mit einem gegen Methyl unempfindlichen Restriktionsenzym, um ein Target-Polynucleotid zu erzeugen, das eine Vielzahl von Cytosinresten umfasst, wobei ein oder mehrere der Cytosinreste methyliert sind;
Ligieren eines Endes des Target-Polynucleotids an ein Eindeutiges Molekulares Identifikator- (UMI-) Polynucleotid und des anderen Endes des Target-Polynucleotids an ein schützendes Polynucleotid, um ein ligiertes Polynucleotid zu bilden, wobei ein Teil des UMI-Polynucleotids eine erste willkürlich erzeugte UMI-Polynucleotidsequenz umfasst, wobei alle der Cytosinreste des UMI-Polynucleotids unmethyliert sind und wobei das schützende Polynucleotid einen exonucleaseresistenten Anteil einschließt, Kontaktieren der Probe mit einer oder mehreren Exonucleasen, die zum Digerieren irgendwelcher Polynucleotide in der Probe, die den exonucleaseresistenten Anteil nicht einschließen, geeignet sind;
Bilden eines umgewandelten Polynucleotids durch chemisches und/oder enzymatisches Umwandeln jedes methylierten Cytosins in dem ersten ligierten Polynucleotid zu Dihydrouracil;
Amplifizieren des umgewandelten Polynucleotids, um eine Vielzahl von Amplikonpolynucleotiden erzeugen,
Sequenzieren der Vielzahl von Amplikonpolynucleotiden, um eine Vielzahl von Amplikonsequenz-Reads zu erzeugen, wobei jedes von den Amplikonsequenz-Reads:
der Polynucleotidsequenz eines der Vielzahl von Amplikonpolynucleotiden entspricht,
die willkürlich erzeugte UMI-Polynucleotidsequenz einschließt, und
ein Thymin an jeder Nucleotidposition, die der Nucleotidposition eines methylierten Cytosins in dem Target-Polynucleotid entspricht, und ein Cytosin an jeder Nucleotidposition, die einem unmethylierten Cytosin in dem Target-Polynucleotid entspricht, einschließt, es sei denn ein Umwandlungsfehler während des Umwandlungsschritts, ein Amplifikationsfehler während des Amplifikationsschritts oder ein Sequenzierfehler während des Sequenzierschritts verursacht, dass das Amplikonsequenz-Read ein Nucleotid einschließt, bei dem es sich nicht um Thymin an einer Nucleotidposition handelt, die der Nucleotidposition eines methylierten Cytosins in dem Target-Polynucleotid entspricht oder ein Nucleotid einschließt, bei dem es sich nicht um Cytosin an einer Nucleotidposition handelt, die der Nucleotidposition eines unmethylierten Cytosins in der Target-Polynucleotid entspricht; und
Ausrichten der Vielzahl von Amplikonsequenz-Reads auf eine Target-Polynucleotid-Referenzsequenz;
wenn der Sequenzierschritt mindestens fünf Amplikonsequenz-Reads erzeugt hat, dann Erzeugen einer Konsens-Polynucleotidsequenz, die der Polynucleotidsequenz des Target-Polynucleotids entspricht, wobei das Erzeugen der Konsens-Polynucleotidsequenz Folgendes umfasst:
Identifizieren jedes 5'-C-G-3'-Nucleotidpaars in der Target-Polynucleotid-Referenzsequenz,
Vergleichen jedes Amplikonsequenz-Reads mit der Target-Polynucleotid-Referenzsequenz, um die Identität jedes Nucleotids in jedem Amplikonsequenzier-Read zu bestimmen, das auf jedes Cytosin in jedem 5'-C-G-3'-Nucleotidpaar in der Target-Polynucleotid-Referenzsequenz ausgerichtet ist,
wenn 50 % oder mehr der Amplikonsequenzier-Reads ein Thymin an einer Position einschließen, die auf ein Cytosin in einem 5'-C-G-3'-Nucleotidpaar in der Target-Polynucleotid-Referenzsequenz ausgerichtet ist, dann Zuweisen eines methylierten Cytosins zu der entsprechenden Position in der Konsens-Polynucleotidsequenz; und
wenn weniger als 50 % der Amplikonsequenzier-Reads ein Thymin an einer Position einschließen, die auf ein Cytosin in einem 5'-C-G-3'-Nucleotidpaar in der Target-Polynucleotid-Referenzsequenz ausgerichtet ist, dann Zuweisen eines unmethylierten Cytosins zu der entsprechenden Position in der Konsens-Polynucleotidsequenz; und
wobei, wenn mindestens 50 % der 5'-C-G-3'-Nucleotidpaare in der Konsens-Polynucleotidsequenz ein methyliertes Cytosin zugewiesen worden ist, dann Designieren des Target-Polynucleotids als methyliert, und wobei, wenn weniger als 50 % der 5'-C-G-3'-Nucleotidpaare in der Konsens-Polynucleotidsequenz ein methyliertes Cytosin zugewiesen worden ist, dann Designieren des Target-Polynucleotids als unmethyliert.

3. Verfahren nach Anspruch 1 oder 2, wobei das Verfahren ferner das Berechnen des Bruchteils von methylierten Target-Polynucleotiden in der Probe durch Teilen der Anzahl von methylierten Target-Polynucleotiden durch die Gesamtanzahl von Target-Polynucleotiden, die in der Probe detektiert worden sind, umfasst.

4. Verfahren nach Anspruch 1 oder 2, wobei die exonucleaseresistente Modifikation eine Phosphorthioatbindung und/oder einen 3-Kohlenstoffabstandhalter umfasst.

5. Verfahren nach entweder Anspruch 1 oder 2 oder 3 oder 4, wobei Ligieren des Target-Polynucleotids an das UMI-Polynucleotid das Anlagern eines ersten Patch-Polynucleotids an sowohl das Target-Polynucleotid als auch das UMI-Polynucleotid umfasst und wobei Ligieren des Target-Polynucleotids an das schützende Polynucleotid das Anlagern eines zweiten Patch-Polynucleotids sowohl an das Target-Polynucleotid als auch das schützende Polynucleotid umfasst.

6. Verfahren nach einem der Ansprüche 1-5, wobei TET2 und APOBEC zum enzymatischen Umwandeln des ligierten Polynucleotids zu dem umgewandelten Polynucleotid verwendet werden.

7. Verfahren nach einem der Ansprüche 1-6, wobei das umgewandelte Polynucleotid unter Anwendung der Polymerasekettenreaktion (PCR) amplifiziert wird.

8. Verfahren nach einem der Ansprüche 1-7, wobei das Target-Polynucleotid aus einer Region eines Genoms stammt, von dem bekannt ist, dass es in einem spezifischen Zelltyp methyliert ist, wobei wahlweise der spezifische Zelltyp ein spezifischer Typ von Krebszelle ist, wobei weiter wahlweise der spezifische Typ von Krebszelle eine Krebszelle ist, die aus der Gruppe ausgewählt ist bestehend aus Brustkrebs, Ovarialkrebs, Lungenkrebs, Bauchspeicheldrüsenkrebs, Kolorektalkrebs, Prostatakrebs, Gebärmutterkrebs, Blasenkrebs und Leberkrebs.

9. Verfahren nach einem der Ansprüche 1 bis 8, wobei Erzeugen der Konsens-Polynucleotidsequenz Zuweisen eines methylierten Cytosins zu der entsprechenden Position in der Konsens-Polynucleotidsequenz umfasst, wenn 90 % oder mehr der Amplikonsequenzier-Reads ein Cytosin an einer Position einschließen, die auf ein Cytosin in einem 5'-C-G-3'-Nucleotidpaar in der Target-Polynucleotid-Referenzsequenz ausgerichtet ist, und Zuweisen eines unmethylierten Cytosins zu der entsprechenden Position in der Konsens-Polynucleotidsequenz, wenn weniger als 90 % des Amplikonsequenzier-Reads ein Cytosin an einer Position einschließen, die auf ein Cytosin in einem 5'-C-G-3'-Nucleotidpaar in der Target-Polynucleotid-Referenzsequenz ausgerichtet ist.

10. Verfahren nach einem der Ansprüche 1 bis 8, wobei das Target-Polynucleotid als methyliert designiert ist, wenn mindestens 75 % der 5'-C-G-3'-Nucleotidpaare in der Konsens-Polynucleotidsequenz ein methyliertes Cytosin zugeordnet worden ist und das Target-Polynucleotid als unmethyliert designiert ist, wenn weniger als 75 % der 5'-C-G-3'-Nucleotidpaare in der Konsens-Polynucleotidsequenz ein methyliertes Cytosin zugeordnet worden ist.

11. Verfahren nach einem der Ansprüche 1 bis 10, wobei das Konsens-Polynucleotid eine Vielzahl von 5'-C-G-3'-Nucleotidpaaren umfasst.

12. Verfahren nach einem der Ansprüche 1-11, wobei die Methylierung von Cytosinresten in einer Vielzahl von Target-Polynucleotidsequenzen in derselben Probe detektiert wird, wobei wahlweise die Vielzahl von Target-Polynucleotiden mehr als zwei Target-Polynucleotide und weniger als 10 000 Target-Polynucleotide umfasst.

13. Verfahren zum Diagnostizieren eines Patienten mit Krebs, wobei das Verfahren das Detektieren der Methylierung von Cytosinresten in einem Target-Polynucleotid in einer Probe von dem Patienten umfasst, wobei die Methylierung von Cytosinresten dem Verfahren nach einem der Ansprüche 1 bis 12 entsprechend detektiert wird und wobei der Patient mit Krebs diagnostiziert wird, wenn Methylierung von Cytosinresten in einem Target-Polynucleotid in der Probe von dem Patienten detektiert wird.

14. Verfahren nach Anspruch 13, wobei der Krebs aus der Gruppe ausgewählt ist bestehend aus Brustkrebs, Ovarialkrebs, Lungenkrebs, Bauchspeicheldrüsenkrebs, Kolorektalkrebs, Prostatakrebs, Gebärmutterkrebs, Blasenkrebs und Leberkrebs.

15. Verfahren nach Anspruch 13 oder 14, wobei das Verfahren ferner anzeigt, wie der Patient, der mit Krebs diagnostiziert worden ist, durch Chemotherapie, Strahlung, Immuntherapie, chirurgischer Resektion oder eine Kombination davon behandelt werden soll.

## Revendications

1. Procédé de détection de la méthylation de résidus de cytosine dans un polynucléotide cible dans un échantillon comprenant de l'ADN, le procédé comprenant :
la digestion de l'ADN dans l'échantillon avec une enzyme de restriction insensible au méthyle pour créer un polynucléotide cible comprenant une pluralité de résidus de cytosine, dans lequel un ou plusieurs des résidus de cytosine sont méthylés ;
la ligature d'une extrémité du polynucléotide cible à un polynucléotide avec identifiant moléculaire unique (UMI) et de l'autre extrémité du polynucléotide cible à un polynucléotide protecteur pour former un polynucléotide ligaturé, dans lequel une partie du polynucléotide UMI comprend une première séquence de polynucléotide UMI générée aléatoirement, dans lequel la totalité des résidus de cytosine du polynucléotide UMI est méthylée, et dans lequel le polynucléotide protecteur inclut une fraction résistante aux exonucléases ;
la mise en contact de l'échantillon avec une ou plusieurs exonucléases adaptées pour digérer l'un quelconque des polynucléotides dans l'échantillon qui n'incluent pas la fraction résistante aux exonucléases ;
la formation d'un polynucléotide converti en convertissant par voie chimique et/ou enzymatique chaque cytosine non méthylée dans le polynucléotide ligaturé en uracile ;
l'amplification du polynucléotide converti pour générer une pluralité de polynucléotides amplicons ;
le séquençage de la pluralité de polynucléotides amplicons pour générer une pluralité de lectures de séquence d'amplicon, dans lequel chacune des lectures de séquence d'amplicon :
correspond à la séquence de polynucléotide de l'un de la pluralité de polynucléotides amplicons ;
inclut la séquence de polynucléotide UMI générée aléatoirement ; et
inclut une thymine à chaque position de nucléotide correspondant à la position de nucléotide d'une cytosine non méthylée dans le polynucléotide cible et une cytosine à chaque position de nucléotide correspondant à une cytosine méthylée dans le polynucléotide cible à moins qu'une erreur de conversion pendant l'étape de conversion, une erreur d'amplification pendant l'étape d'amplification ou une erreur de séquençage pendant l'étape de séquençage n'amène la lecture de séquence d'amplicon à inclure un nucléotide autre que la thymine à une position de nucléotide correspondant à la position de nucléotide d'une cytosine non méthylée dans le polynucléotide cible ou à inclure un nucléotide autre que la cytosine à une position de nucléotide correspondant à la position de nucléotide d'une cytosine méthylée dans le polynucléotide cible ; et
l'alignement de la pluralité de lectures de séquençage d'amplicon sur une séquence de référence de polynucléotide cible ;
si l'étape de séquençage a généré au moins cinq lectures de séquence d'amplicon, la génération d'une séquence de polynucléotide consensus correspondant à la séquence de polynucléotide du polynucléotide cible, dans lequel la génération de la séquence de polynucléotide consensus comprend :
l'identification de chaque paire de nucléotides 5'-C-G-3' dans la séquence de référence de polynucléotide cible ;
la comparaison de chaque lecture de séquençage d'amplicon avec la séquence de référence de polynucléotide cible pour déterminer l'identité de chaque nucléotide dans chaque lecture de séquençage d'amplicon aligné sur chaque cytosine dans chaque paire de nucléotides 5'-C-G-3' dans la séquence de référence de polynucléotide cible ;
si 50 % ou plus des lectures de séquençage d'amplicon incluent une cytosine en une position alignée sur une cytosine dans une paire de nucléotides 5'-C-G-3' dans la séquence de référence de polynucléotide cible, l'attribution d'une cytosine méthylée à la position correspondante dans la séquence de polynucléotide consensus ; et
si moins de 50 % des lectures de séquençage d'amplicon incluent une cytosine en une position alignée sur une cytosine dans une paire de nucléotides 5'-C-G-3' dans la séquence de référence de polynucléotide cible, l'attribution d'une cytosine non méthylée à la position correspondante dans la séquence de polynucléotide consensus ; et
dans lequel si au moins 50 % des paires de nucléotides 5'-C-G-3' dans la séquence de polynucléotide consensus se sont vus attribuer une cytosine méthylée, la désignation du polynucléotide cible comme méthylé, et dans lequel si moins de 50 % des paires de nucléotides 5'-C-G-3' dans la séquence de polynucléotide consensus se sont vus attribuer une cytosine méthylée, la désignation du polynucléotide cible comme non méthylé.

2. Procédé de détection de la méthylation de résidus de cytosine dans un polynucléotide cible dans un échantillon comprenant de l'ADN, le procédé comprenant :
la digestion de l'ADN dans l'échantillon avec une enzyme de restriction insensible au méthyle pour créer un polynucléotide cible comprenant une pluralité de résidus de cytosine, dans lequel un ou plusieurs des résidus de cytosine sont méthylés ;
la ligature d'une extrémité du polynucléotide cible à un polynucléotide avec identifiant moléculaire unique (UMI) et de l'autre extrémité du polynucléotide cible à un polynucléotide protecteur pour former un polynucléotide ligaturé, dans lequel une partie du polynucléotide UMI comprend une première séquence de polynucléotide UMI générée aléatoirement, dans lequel la totalité des résidus de cytosine du polynucléotide UMI est non méthylée, et dans lequel le polynucléotide protecteur inclut une fraction résistante aux exonucléases ; la mise en contact de l'échantillon avec une ou plusieurs exonucléases adaptées pour digérer l'un quelconque des polynucléotides dans l'échantillon qui n'incluent pas la fraction résistante aux exonucléases ;
la formation d'un polynucléotide converti en convertissant par voie chimique et/ou enzymatique chaque cytosine méthylée dans le premier polynucléotide ligaturé en dihydro-uracile ;
l'amplification du polynucléotide converti pour générer une pluralité de polynucléotides amplicons ;
le séquençage de la pluralité de polynucléotides amplicons pour générer une pluralité de lectures de séquence d'amplicon, dans lequel chacune des lectures de séquence d'amplicon :
correspond à la séquence de polynucléotide de l'un de la pluralité de polynucléotides amplicons ;
inclut la séquence de polynucléotide UMI générée aléatoirement ; et
inclut une thymine à chaque position de nucléotide correspondant à la position de nucléotide d'une cytosine méthylée dans le polynucléotide cible et une cytosine à chaque position de nucléotide correspondant à une cytosine non méthylée dans le polynucléotide cible à moins qu'une erreur de conversion pendant l'étape de conversion, une erreur d'amplification pendant l'étape d'amplification ou une erreur de séquençage pendant l'étape de séquençage n'amène la lecture de séquence d'amplicon à inclure un nucléotide autre que la thymine à une position de nucléotide correspondant à la position de nucléotide d'une cytosine méthylée dans le polynucléotide cible ou à inclure un nucléotide autre que la cytosine à une position de nucléotide correspondant à la position de nucléotide d'une cytosine non méthylée dans le polynucléotide cible ; et
l'alignement de la pluralité de lectures de séquençage d'amplicon sur une séquence de référence de polynucléotide cible ;
si l'étape de séquençage a généré au moins cinq lectures de séquence d'amplicon, la génération d'une séquence de polynucléotide consensus correspondant à la séquence de polynucléotide du polynucléotide cible, dans lequel la génération de la séquence de polynucléotide consensus comprend :
l'identification de chaque paire de nucléotides 5'-C-G-3' dans la séquence de référence de polynucléotide cible ;
la comparaison de chaque lecture de séquençage d'amplicon avec la séquence de référence de polynucléotide cible pour déterminer l'identité de chaque nucléotide dans chaque lecture de séquençage d'amplicon alignée sur chaque cytosine dans chaque paire de nucléotides 5'-C-G-3' dans la séquence de référence de polynucléotide cible ;
si 50 % ou plus des lectures de séquençage d'amplicon incluent une thymine en une position alignée sur une cytosine dans une paire de nucléotides 5'-C-G-3' dans la séquence de référence de polynucléotide cible, l'attribution d'une cytosine méthylée à la position correspondante dans la séquence de polynucléotide consensus ; et
si moins de 50 % des lectures de séquençage d'amplicon incluent une thymine en une position alignée sur une cytosine dans une paire de nucléotides 5'-C-G-3' dans la séquence de référence de polynucléotide cible, l'attribution d'une cytosine non méthylée à la position correspondante dans la séquence de polynucléotide consensus ; et
dans lequel si au moins 50 % des paires de nucléotides 5'-C-G-3' dans la séquence de polynucléotide consensus se sont vus attribuer une cytosine méthylée, la désignation du polynucléotide cible comme méthylé, et dans lequel si moins de 50 % des paires de nucléotides 5'-C-G-3' dans la séquence de polynucléotide consensus se sont vus attribuer une cytosine méthylée, la désignation du polynucléotide cible comme non méthylé.

3. Procédé selon les revendications 1 ou 2, le procédé comprenant en outre le calcul de la fraction de polynucléotides cibles méthylés dans l'échantillon en divisant le nombre de polynucléotides cibles méthylés par le nombre total de polynucléotides cibles détectés dans l'échantillon.

4. Procédé selon les revendications 1 ou 2, dans lequel la modification résistante aux exonucléases comprend une liaison phosphorothioate et/ou un espaceur de carbone 3.

5. Procédé selon l'une des revendications 1 ou 2 ou 3 ou 4, dans lequel la ligature du polynucléotide cible au polynucléotide UMI comprend l'annelage d'un premier polynucléotide patch à la fois au polynucléotide cible et au polynucléotide UMI, et dans lequel la ligature du polynucléotide cible au polynucléotide protecteur comprend l'annelage d'un deuxième polynucléotide patch à la fois au polynucléotide cible et au polynucléotide protecteur.

6. Procédé selon l'une quelconque des revendications 1 à 5, dans lequel TET2 et APOBEC sont utilisés pour convertir par voie enzymatique le polynucléotide ligaturé en polynucléotide converti.

7. Procédé selon l'une quelconque des revendications 1 à 6, dans lequel le polynucléotide converti est amplifié en utilisant l'amplification en chaîne par polymérase (ACP).

8. Procédé selon l'une quelconque des revendications 1 à 7, dans lequel le polynucléotide cible vient d'une région d'un génome connue pour être méthylée dans un type de cellule spécifique, facultativement dans lequel le type de cellule spécifique est un type spécifique de cellule cancéreuse, plus facultativement dans lequel le type spécifique de cellule cancéreuse est une cellule cancéreuse choisie dans le groupe constitué par le cancer du sein, le cancer de l'ovaire, le cancer du poumon, le cancer du pancréas, le cancer colorectal, le cancer de la prostate, le cancer de l'utérus, le cancer de la vessie et le cancer du foie.

9. Procédé selon l'une quelconque des revendications 1 à 8, dans lequel la génération de la séquence de polynucléotide consensus comprend l'attribution d'une cytosine méthylée à la position correspondante dans la séquence de polynucléotide consensus si 90 % ou plus des lectures de séquençage d'amplicon incluent une cytosine à une position alignée sur une cytosine dans une paire de nucléotides 5'-C-G-3' dans la séquence de référence de polynucléotide cible, et l'attribution d'une cytosine non méthylée à la position correspondante dans la séquence de polynucléotide consensus si moins de 90 % des lectures de séquençage d'amplicon incluent une cytosine à une position alignée sur une cytosine dans une paire de nucléotides 5'-C-G-3' dans la séquence de référence de polynucléotide cible.

10. Procédé selon l'une quelconque des revendications 1 à 8, dans lequel le polynucléotide cible est désigné comme méthylé si au moins 75 % des paires de nucléotides 5'-C-G-3' dans la séquence de polynucléotide consensus se sont vus attribuer une cytosine méthylée, et le polynucléotide cible est désigné comme non méthylé si moins de 75 % des paires de nucléotides 5'-C-G-3' dans la séquence de polynucléotide consensus se sont vus attribuer une cytosine méthylée.

11. Procédé selon l'une quelconque des revendications 1 à 10, dans lequel le polynucléotide consensus comprend une pluralité de paires de nucléotides 5'-C-G-3'.

12. Procédé selon l'une quelconque des revendications 1 à 11, dans lequel la méthylation de résidus de cytosine dans une pluralité de séquences de polynucléotide cible est détectée dans le même échantillon, facultativement, dans lequel la pluralité de polynucléotides cibles comprend plus de deux polynucléotides cibles et moins de 10 000 polynucléotides cibles.

13. Procédé de diagnostic d'un cancer chez un patient, le procédé comprenant la détection de la méthylation de résidus de cytosine dans un polynucléotide cible dans un échantillon du patient, dans lequel la méthylation de résidus de cytosine est détectée conformément au procédé selon l'une quelconque des revendications 1 à 12 et dans lequel un cancer est diagnostiqué chez le patient lorsque la méthylation de résidus de cytosine dans un polynucléotide cible est détectée dans l'échantillon du patient.

14. Procédé selon la revendication 13, dans lequel le cancer est choisi dans le groupe constitué par un cancer du sein, un cancer de l'ovaire, un cancer du poumon, un cancer du pancréas, un cancer colorectal, un cancer de la prostate, un cancer de l'utérus, un cancer de la vessie et un cancer du foie.

15. Procédé selon la revendication 13 ou 14, le procédé indiquant en outre comment traiter le patient, chez qui un cancer a été diagnostiqué, par chimiothérapie, rayonnement, immunothérapie, résection chirurgicale ou une combinaison de ceux-ci.
